# EUROPEAN PATENT SPECIFICATION

(11) **EP 2 346 329 B1**
(45) Date of publication and mention of the grant of the patent: **21.08.2013**
(21) Application number: 09819961.5
(22) Date of filing: 09.10.2009
(51) Int. Cl.: A01N 41/06, A61K 31/18, A61P 31/14

(54) **A METHOD OF INHIBITING HEPATITIS C VIRUS BY COMBINATION OF A 5,6-DIHYDRO-1H-PYRIDIN-2-ONE AND ONE OR MORE ADDITIONAL ANTIVIRAL COMPOUNDS**
VERFAHREN ZUR HEMMUNG DES HEPATITIS-C-VIRUS MITTELS KOMBINATION AUS EINEM 5,6-DIHYDRO-1H-PYRIDIN-2-ON UND EINER ODER MEHREREN ANTIVIRALEN VERBINDUNGEN
PROCÉDÉ D'INHIBITION DU VIRUS DE L'HÉPATITE C PAR COMBINAISON D'UNE 5,6-DIHYDRO-1H-PYRIDIN-2-ONE ET D'UN OU PLUSIEURS COMPOSÉS ANTIVIRAUX SUPPLÉMENTAIRES

(30) Priority: 09.10.2008 US 104152 P
(43) Date of publication of application: 27.07.2011
(73) Proprietor: Anadys Pharmaceuticals, Inc., San Diego, California 92121 (US)
(72) Inventor: DRAGOVICH, Peter, S., San Mateo CA 94403 (US); THOMPSON, Peggy, A., San Diego CA 92103 (US); RUEBSAM, Frank, San Diego CA 92116 (US)
(74) Representative: Schiweck, Weinzierl & Koch
(86) International application number: PCT/US2009/060189
(87) International publication number: WO 2010/042834

(56) References cited:
- WO-A1-2008/124450
- WO-A1-2009/008989
- US-A1- 2005 107 364
- US-A1- 2008 050 336
- US-A1- 2008 214 529
- US-A1- 2008 292 588

## Description

### FIELD OF THE INVENTION

The invention is directed to a method of treating infections by hepatitis C virus by administering *N*-{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide and one or more additional antiviral compounds or pharmaceutical compositions containing such compounds.

### BACKGROUND OF THE INVENTION

Hepatitis C is a major health problem world-wide. The World Health Organization estimates that 170 million people are chronic carriers of the hepatitis C virus (HCV), with 4 million carriers in the United States alone. In the United States, HCV infection accounts for 40% of chronic liver disease and HCV disease is the most common cause for liver transplantation. HCV infection leads to a chronic infection and about 70% of persons infected will develop chronic histological changes in the liver (chronic hepatitis) with a 10-40% risk of cirrhosis and an estimated 4% lifetime risk of hepatocellular carcinoma. The CDC estimates that each year in the United States there are 35,000 new cases of HCV infection and approximately ten thousand deaths attributed to HCV disease.

The current standard of care is a pegylated interferon/ribavirin combination at a cost of approximately $30,000/year. These drugs have difficult dosing problems and side-effects and do not achieve a sustained virological response in a significant number of diagnosed patients. Pegylated interferon treatment is associated with menacing flu-like symptoms, irritability, inability to concentrate, suicidal ideation, and leukocytopenia. Ribavirin is associated with hemolytic anemia and birth defects.

The overall response to this standard therapy is low; as approximately one third of patients do not respond. Of those who do respond, some relapse within six months of completing 6-12 months of therapy. As a consequence, the long-term response rate for all patients entering treatment is only about 50%. The relatively low response rate and the significant side-effects of current therapy anti-HCV drug treatments, coupled with the negative long term effects of chronic HCV infection, result in a continuing medical need for improved therapy. Antiviral pharmaceuticals to treat RNA virus diseases like HCV are few, and as described above are often associated with multiple adverse effects.

A number of publications have described NS5B inhibitors useful in the treatment of hepatitis C infection. *See, e.g.,* International Publication No. WO 2008/124450 (disclosing certain 5,6-dihydro-1H-pyridin-2-one compounds); U.S. Patent Application Publication No. US 2008/0031852 (describing [1,2-*b*] pyridazinone compounds); U.S. Patent Application Publication No. US 2006/0189602 (disclosing certain pyridazinones); U.S. Patent Application Publication No. US 2006/0252785 (disclosing selected heterocyclics); and International Publication Nos. WO 03/059356, WO 2002/098424, and WO 01/85172 (each describing a particular class of substituted thiadiazines).
While there are, in some cases, medicines available to reduce disease symptoms, there are few drugs to effectively inhibit replication of the underlying virus. The significance and prevalence of RNA virus diseases, including but not limited to chronic infection by the hepatitis C virus, and coupled with the limited availability and effectiveness of current antiviral pharmaceuticals, have created a compelling and continuing need for new pharmaceuticals to treat these diseases.

The genetic heterogeneity or quasispecies nature of HCV has important implications for therapy. This profound genetic mutability can allow the virus to escape the antiviral pressure exerted by treatment with any single direct antiviral agent. Indeed, selection of mutations conferring resistance to either NS3 serine protease inhibitors or to NS5B RNA-dependent polymerase inhibitors both *in vitro* and *in vivo* have been described in the literature. Drug resistance can be minimized by the appropriate use of combinations of agents which have a low probability of cross-resistance. Examples include the combination of non-nucleoside inhibitors of the viral polymerase with inhibitors of the NS3 serine protease and / or nucleoside inhibitors of the viral polymerase. Furthermore, mutations in the virus that confer resistance to direct antiviral agents do not appear to affect sensitivity to interferon. It is therefore anticipated that in the near future, treatment of HCV, especially genotype I, will consist of an extended course of administration of appropriately selected combinations of agents.
International Patent Application WO 2009/008989 A1 discloses a compound of Formula 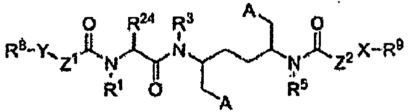 or a pharmaceutically acceptable salt, solvate, and/or ester thereof, compositions containing such compounds, as well as therapeutic methods that include the administration of such compounds. In this formula Z¹ and Z² are each independently -O- or -N(R⁷)-; X and Y are each independently aryl, arylalkyl, heterocyclyl, or heterocyclylalkyl; R¹, R³, and R⁵ are each independently selected from the group consisting of H, alkyl, substituted alkyl, arylalkyl, and substituted arylalkyl; each R⁷ is independently selected from the group consisting of H, alkyl, substituted alkyl, heteroalkyl, substituted heteroalkyl, carbocyclyl, substituted carbocyclyl, heterocyclyl, and substituted heterocyclyl; R⁸ and R⁹ are each one or more substituents independently selected from the group consisting of H⁷ alkyl, halogen, aryl, heterocyclyl, and CN; and R²⁴ is selected from the group consisting of -alkylene-NR5-C(O)-R²⁵, -alkylene-C(O)-NR⁵-R²⁶, -alkylene-N(R²⁷)₂,-CH₂-heterocyclyl, and substituted-CH₂-heterocyclyl; R²⁵ is alkyl, heterocyclylalkyl, or substituted heterocyclylalkyl; R²⁶ is a substituted or unsubstituted heterocyclyl; or R5 and R²⁶ together with the nitrogen atom to which they are both shown attached, form a substituted or unsubstituted bicyclic
heterocyclyl; each R²⁷ is independently a substituted alkyl which may be the same or different; or each R²⁷, together with the nitrogen atom to which they are both shown attached, form a substituted or unsubstituted bicyclic heterocyclyl.

### SUMMARY OF THE INVENTION

The present invention describes an *in vitro* method of inhibiting hepatitis C virus replication comprising exposing hepatitis C virus to a therapeutically effective amount of a composition comprising *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, or a salt or hydrate thereof, and a composition comprising one or more additional antiviral compounds selected from the group consisting of: VBY-376, BMS-650032, MK-7009, TMC-435350, BI-201335, GS-9190, MK-3281, VCH-759 (VX-759), VCH-916, ABT-333, BMS-791325, PF-00868554 (filibuvir), IDX-184, RG7128, PSI-6130, BMS-790052, and ANA773..

In one embodiment, the method comprises exposing the virus to the compositions separately or together, or to the combined compositions.

In one embodiment, the hepatitis C virus is in a human liver cell.

In another aspect, a combination for use in treating or preventing hepatitis C virus infection is disclosed. The combination for use comprises a composition comprising *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, or a salt or hydrate thereof, and a composition comprising one or more additional antiviral compounds selected from the group consisting of: VBY-376, BMS-650032, MK-7009, TMC-435350, BI-201335, GS-9190, MK-3281, VCH-759 (VX-759), VCH-916, ABT-333, BMS-791325, PF-00868554 (filibuvir), IDX-184, RG7128, PSI-6130, BMS-790052, ANA773, SCH900518 (narlaprevir), VX-813, VX-985, PHX1766, ABT-450, ACH-1625, ACH-1095, IDX136, IDX316, ITMN-5489, PSI-7851, VCH-222 (VX-222), ABT-072, BI207127, Debio-025, NIM-811, SCY-635, AZD2836, BMS-824393, PF-04878691, Locteron, Omega interferon, PEG-Interferon lambda, GI-5005, taribavirin, VX-950 (telapravir), SCH-503034 (boceprevir), Interferon α-2a, and IMO-2125.

The compositions of the combination for use may be administered separately (e.g. temporally or spatially) or together, or to the combined compositions.

The combination may be for use in a mammal such as a human.

In yet another aspect, a pharmaceutically acceptable composition comprising a therapeutically effective amount of a composition comprising *N-*{3-[{1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, or a salt or hydrate thereof, and one or more additional antiviral compounds selected from the group consisting of: VBY-376, BMS-650032, MK-7009, TMC-435350, BI-201335, GS-9190, MK-3281, VCH-759 (VX-759), VCH-916, ABT-333, BMS-791325, PF-00868554 (filibuvir), IDX-184, RG7128, PSI-6130, BMS-790052, ANA773, SCH900518 (narlaprevir), VX-813, VX-985, PHX1766, ABT-450, ACH-1625, ACH-1095, IDX136, IDX316, ITMN-5489, PSI-7851, VCH-222 (VX-222), ABT-072, BI207127, Debio-025, NIM-811, SCY-635, AZD2836, BMS-824393, PF-04878691, BLX-833 (Locteron), Omega interferon, PEG-Interferon lambda, taribavirin, and IMO-2125, and a pharmaceutically acceptable carrier is disclosed.

In an embodiment, the one or more additional antiviral compounds are selected from MK-7009, TMC-435350, BI-201335, PF-00868554 (filibuvir), IDX-184, RG7128, PSI-6130, BMS-790052, and ANA773.

Disclosed are also compositions comprising at least two antiviral agents, one of which is selected from the group consisting of:
*N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide,
*N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, L-arginine salt,
*N*-{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, *L*-lysine salt,
*N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, hemi magnesium salt,
*N*-{3-[(1*R*,2*S*,7*R*,8*S*-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, sodium salt, and
*N*-{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro- 1λ⁶-benzo[1,2,4]thiadiazin-7-yl}-methanesulfonamide, potassium salt.

### BRIEF DESCRIPTION OF THE DRAWINGS

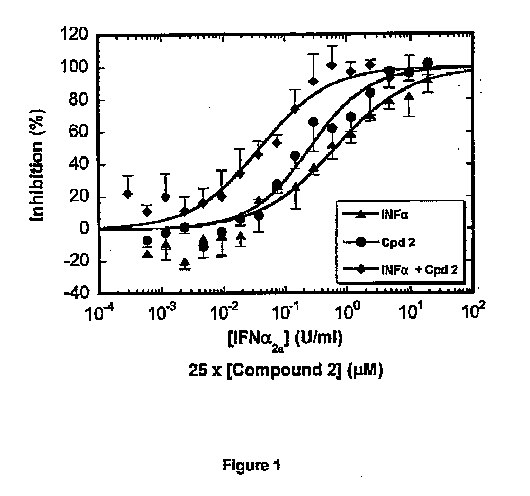

Figure 1 shows the EC₅₀ of *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (Compound 2) and Interferon α-2a (IFN) tested alone and in combination.

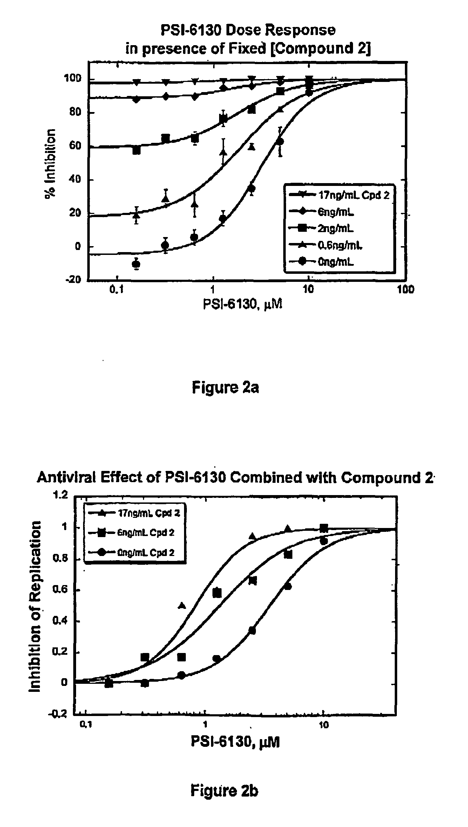

Figures 2a and 2b show the dose response of the antiviral agent PSI-6130 evaluated in the presence of fixed concentrations of *N-*{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (Compound 2).

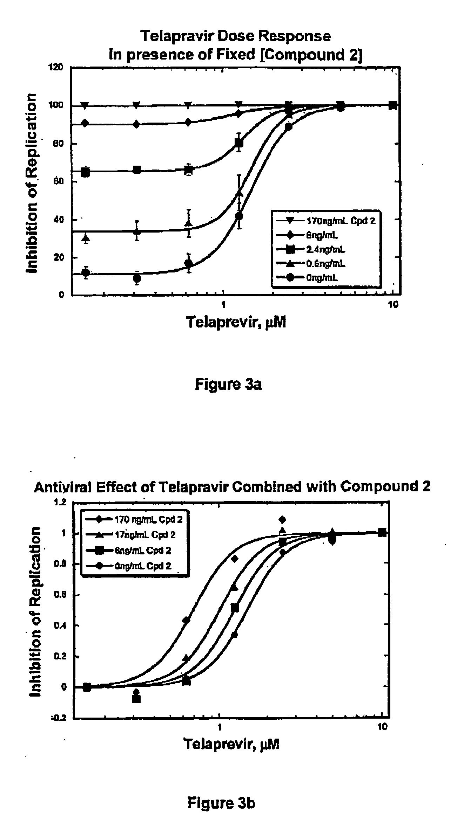

Figures 3a and 3b show the dose response of the antiviral agent Telapravir evaluated in the presence fixed concentrations of *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (Compound **2)**.

### DETAILED DESCRIPTION OF THE INVENTION

Where the following terms are used in this specification, they are used as defined below:

The terms "comprising," "having" and "including" are used herein in their open, non-limiting sense.

The term "immunomodulator" refers to natural or synthetic products capable of modifying the normal or aberrant immune system through stimulation or suppression.

The term "preventing" refers to the ability of a compound or composition of the invention to prevent a disease identified herein in patients diagnosed as having the disease or who are at risk of developing such disease. The term also encompasses preventing further progression of the disease in patients who are already suffering from or have symptoms of such disease.

The term "patient" or "subject" means an animal (e.g., cow, horse, sheep, pig, chicken, turkey, quail, cat, dog, mouse, rat, rabbit, guinea pig, etc.) or a mammal, including chimeric and transgenic animals and mammals. In the treatment or prevention of HCV infection, the term "patient" or "subject" preferably means a monkey or a human, most preferably a human. In a specific embodiment the patient or subject is infected by or exposed to the hepatitis C virus. In certain embodiments, the patient is a human infant (age 0-2), child (age 2-17), adolescent (age 12-17), adult (age 18 and up) or geriatric (age 70 and up) patient. In addition, the patient includes immunocompromised patients such as HIV positive patients, cancer patients, patients undergoing immunotherapy or chemotherapy. In a particular embodiment, the patient is a healthy individual, i.e., not displaying symptoms of other viral infections.

The term a "therapeutically effective amount" refers to an amount of the compound of the invention sufficient to provide a benefit in the treatment or prevention of viral disease, to delay or minimize symptoms associated with viral infection or viral-induced disease, or to cure or ameliorate the disease or infection or cause thereof. In particular, a therapeutically effective amount means an amount sufficient to provide a therapeutic benefit *in vivo.* Used in connection with an amount of a compound of the invention, the term preferably encompasses a non-toxic amount that improves overall therapy, reduces or avoids symptoms or causes of disease, or enhances the therapeutic efficacy of or synergies with another therapeutic agent.

The term a "prophylactically effective amount" refers to an amount of a compound of the invention or other active ingredient sufficient to result in the prevention of infection, recurrence, or spread of viral infection. A prophylactically effective amount may refer to an amount sufficient to prevent initial infection or the recurrence or spread of the infection or a disease associated with the infection. Used in connection with an amount of a compound of the invention, the term preferably encompasses a non-toxic amount that improves overall prophylaxis or enhances the prophylactic efficacy of or synergies with another prophylactic or therapeutic agent.

The term "in combination" refers to the use of more than one prophylactic and/or therapeutic agent simultaneously or sequentially and in a manner that their respective effects are additive or synergistic.

The term "treating" refers to:
(i) preventing a disease, disorder, or condition from occurring in an animal that may be predisposed to the disease, disorder and/or condition, but has not yet been diagnosed as having it;
(ii) inhibiting the disease, disorder, or condition, i.e., arresting its development; and
(iii) relieving the disease, disorder, or condition, i.e., causing regression of the disease, disorder, and/or condition.

The terms "R" and "S" indicate the specific stereochemical configuration of a substituent at an asymmetric carbon atom in a chemical structure as drawn.

The term "rac" indicates that a compound is a racemate, which is defined as an equimolar mixture of a pair of enantiomers. A "rac" compound does not exhibit optical activity. The chemical name or formula of a racemate is distinguished from those of the enantiomers by the prefix (±)- or *rac-* (or *racem-*) or by the symbols *RS* and *SR.*

The terms "endo" and "exo" are descriptors of the relative orientation of substituents attached to non-bridgehead atoms in a bicyclo[*x.y.z*]alkane (*x* ≥ *y* > *z* > 0).

The terms "syn" and "anti" are descriptors of the relative orientation of substituents attached to bridgehead atoms in a bicyclo[*x.y.z*]alkane *(x* ≥ *y* > *z* > 0).

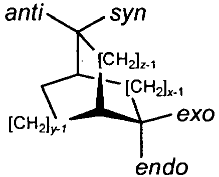

The term "exo" is given to a substituent (e.g., Br attached to C-2 in the example below) that is orientated towards the highest numbered bridge (z bridge, e.g., C-7 in example below); if the substituent is orientated away from the highest numbered bridge it is given the description "endo".

The term "syn" is given to a substituent attached to the highest numbered bridge (z bridge, e.g., F attached to C-7 in the example below) and is orientated towards the lowest numbered bridge (x bridge, e.g., C-2 and C-3 in example below); if the substituent is orientated away from the lowest numbered bridge it is given the description "anti."

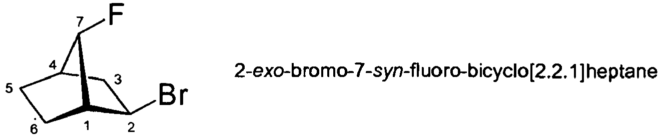

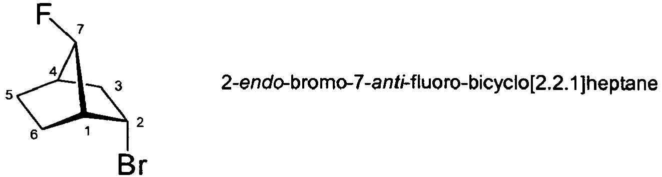

The terms "cis" and "trans" are descriptors which show the relationship between two ligands attached to separate atoms that are connected by a double bond or are contained in a ring. The two ligands are said to be located *cis* to each other if they lie on the same side of a plane. If they are on opposite sides, their relative position is described as *trans.* The appropriate reference plane of a double bond is perpendicular to that of the relevant σ-bonds and passes through the double bond. For a ring it is the mean plane of the ring(s).

As generally understood by those skilled in the art, an optically pure compound having one chiral center (i.e., one asymmetric carbon atom) is one that consists essentially of one of the two possible enantiomers (i.e., is enantiomerically pure), and an optically pure compound having more than one chiral center is one that is both diastereomerically pure and enantiomerically pure. Preferably, the compounds of the present invention are used in a form that is at least 90% free of other enantiomers or diastereomers of the compounds, that is, a form that contains at least 90% of a single isomer (80% enantiomeric excess ("e.e.") or diastereomeric excess ("d.e.")), more preferably at least 95% (90% e.e. or d.e.), even more preferably at least 97.5% (95% e.e. or d.e.), and most preferably at least 99% (98% e.e. or d.e.).

Additionally, the invention is intended to cover solvated as well as unsolvated forms of the identified compounds, and can include both hydrated and non-hydrated forms. Other examples of solvates include the compounds in combination with isopropanol, ethanol, methanol, DMSO, ethyl acetate, pentyl acetate, acetic acid, or ethanolamine.

The invention is also intended to cover deuterated forms of *N-*{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, and pharmaceutically acceptable salts thereof.

In addition to compounds of the invention, the invention includes pharmaceutically acceptable prodrugs, pharmaceutically active metabolites, and pharmaceutically acceptable salts of such compounds and metabolites.

"A pharmaceutically acceptable prodrug" is a compound that may be converted under physiological conditions or by solvolysis to the specified compound or to a pharmaceutically acceptable salt of such compound prior to exhibiting its pharmacological effect (s). Typically, the prodrug is formulated with the objective(s) of improved chemical stability, improved patient acceptance and compliance, improved bioavailability, prolonged duration of action, improved organ selectivity, improved formulation (e.g., increased hydrosolubility), and/or decreased side effects (e.g., toxicity). The prodrug can be readily prepared from the compounds of Formula I using methods known in the art, such as those described by Burger's Medicinal Chemistry and Drug Chemistry, 1, 172-178, 949-982 (1995). See also Bertolini et al., J. Med. Chem., 40, 2011-2016 (1997); Shan, et al., J. Pharm. Sci., 86 (7), 765-767; Bagshawe, Drug Dev. Res., 34, 220-230 (1995); Bodor, Advances in Drug Res., 13, 224-331 (1984); Bundgaard, Design of Prodrugs (Elsevier Press 1985); Larsen, Design and Application of Prodrugs, Drug Design and Development (Krogsgaard-Larsen et al., eds., Harwood Academic Publishers, 1991); Dear et al., J. Chromatogr. B, 748, 281-293 (2000); Spraul et al., J. Pharmaceutical & Biomedical Analysis, 10, 601-605 (1992); and Prox et al., Xenobiol., 3, 103-112 (1992).

"A pharmaceutically active metabolite" is intended to mean a pharmacologically active product produced through metabolism in the body of a specified compound or salt thereof. After entry into the body, most drugs are substrates for chemical reactions that may change their physical properties and biologic effects. These metabolic conversions, which usually affect the polarity of the Formula I compounds, alter the way in which drugs are distributed in and excreted from the body. However, in some cases, metabolism of a drug is required for therapeutic effect. For example, anticancer drugs of the anti-metabolite class must be converted to their active forms after they have been transported into a cancer cell.

Since most drugs undergo metabolic transformation of some kind, the biochemical reactions that play a role in drug metabolism may be numerous and diverse. The main site of drug metabolism is the liver, although other tissues may also participate.

A feature characteristic of many of these transformations is that the metabolic products, or "metabolites," are more polar than the parent drugs, although a polar drug does sometime yield a less polar product. Substances with high lipid/water partition coefficients, which pass easily across membranes, also diffuse back readily from tubular urine through the renal tubular cells into the plasma. Thus, such substances tend to have a low renal clearance and a long persistence in the body. If a drug is metabolized to a more polar compound, one with a lower partition coefficient, its tubular reabsorption will be greatly reduced. Moreover, the specific secretory mechanisms for anions and cations in the proximal renal tubules and in the parenchymal liver cells operate upon highly polar substances.

As a specific example, phenacetin (acetophenetidin) and acetanilide are both mild analgesic and antipyretic agents, but are transformed within the body to a more polar and more effective metabolite, p-hydroxyacetanilid (acetaminophen), which is widely used today. When a dose of acetanilide is given to a person, the successive metabolites peak and decay in the plasma sequentially. During the first hour, acetanilide is the principal plasma component. In the second hour, as the acetanilide level falls, the metabolite acetaminophen concentration reaches a peak Finally, after a few hours, the principal plasma component is a further metabolite that is inert and can be excreted from the body. Thus, the plasma concentrations of one or more metabolites, as well as the drug itself, can be pharmacologically important.

"A pharmaceutically acceptable salt" is intended to mean a salt that retains the biological effectiveness of the free acids and bases of the specified compound and that is not biologically or otherwise undesirable. A compound of the invention may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of the compounds of the present invention with a mineral or organic acid or an inorganic base, such as salts including sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1-sulfonates, naphthalene-2-sulfonates, and mandelates.

If a composition employed in the context of the invention has a base, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid , such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an α-hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If a composition employed in the context of the invention has an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum, and lithium.

In the case of agents that are solids, it is understood by those skilled in the art that the inventive compounds and salts may exist in different crystal, co-crystal, or polymorphic forms, all of which are intended to be within the scope of the present invention and specified formulas.

The anti-viral agents are selected from the group consisting of: VBY-376 (http://clinicaltrials.gov/ct2/show/NCT00557583), BMS-650032 (http://clinicaltrials.gov/ct2/show/NCT00559247), MK-7009 (Lawitz, E. J.; et al., American Association for the Study of Liver Diseases, 59th Annual Meeting, San Francisco, October 31-November 4, 2008; Abstract # 211), TMC-435350 (Raboisson, P.; et al., Bioorg. Med. Chem. Lett., 2008, 18, 4853), BI-201335 (Manns, M. P.; et al., American Association for the Study of Liver Diseases, 59th Annual Meeting, San Francisco, October 31-November 4, 2008; Abstract # 1849), GS-9190 (Yang, C.; et al., Americal Association for the Study of Liver Diseases, 58th Annual Meeting, Boston, November 2-6, 2007; Abstract # 1398), MK-3281 (http://clinicaltrials.gov/ct2/show/NCT00635804), VCH-759 (Cooper, C.; et al., J. Hepatology, 2009, 51, 39), VCH-916 (Proulx, L.; et al. European Association for the Study of the Liver, 43rd Annual Meeting, Milan, Italy, April 23-27, 2008), ABT-333 (http://clinicaltrials.gov/ct2/show/NCT00696904), BMS-791325 (http://clinicaltrials.gov/ct2/show/NCT00664625), PF-00868554 (Shi, S.; et al., Antimicrob. Agents Chemother, 2009, 53, 2544), IDX-184 (Cretton-Scott, E.; et al., European Association for the Study of the Liver, 43rd Annual Meeting, Milan, Italy, April 23-27, 2008; Abstract # 588), RG7128 (Clark, J. L.; et al., J. Med. Chem., 2005, 48, 5504) or its parent compound PSI-6130 (Stuyver, L. J.; et al., Antiviral Chemistry and Chemotherapy, 2006, 17, 79), BMS-790052 (http://clinicaltrials.gov/ct2/show/NCT00546715), and ANA773 (Anadys Pharmaceuticals, company public website (htttp://ir.anadyspharma.com/phoenix.zhtml?c=148908&p=irol-newsArticle&ID=1172413&highlight=)). Disclosed is also the use of SCH900518 (Hughes, Y. et al.; J. Hepatology, 2009, 50, S345), VX-813 (Vertex Pharmaceuticals, company public website: http://www.vrtx.com/current-projects/drug-candidates/vx-813.html), VX-985 (Vertex Pharmaceuticals, company public website: http://www.vrtx.com/current-projects/drug-candidates/vx-985.html), PHX1766 (Phenomix Corp. company public website: http://www.phenomixcorp.com/products-hepatitis_c.asp), ABT-450 (Abbott Laboratory, company public website: http://www.abbott.com/global/url/pressRelease/en US/60.5:5/Press Release 0699.ht m), ACH-1625 (Achillion Pharmaceuticals, company public website: http://www.achillion.com/main.aspx?pn=1625&fl=1), PSI-7851 (Furman, P.A., et al.; 15th International Symposium on HCV & Related Viruses, San Antonio, TX, October 5-9, 2008; Abstract # 275), VCH-222 (Cooper, C. et al.; J. Hepatology, 2009, 50, S342), ABT-072 (Koev, G. et al.; J. Hepatology, 2009, 50, S346), BI207127 (Larrey, D. et al.; J. Hepatology, 2009, 50, S383), Debio-025 (Coelmont, L. et al.; Antimicob. Agents and Chemother., 2009, 53, 967; Herrmann, E. et al.; J. Hepatology, 2009, 50, S344), NIM-811 (Mlynar, E. et al.; J. Gen. Virol., 1997, 78, 825; Lawitz, E. et al.; J. Hepatology, 2009, 50, S379), SCY-635 (Chatterji, U. et al.; J. Biol. Chem., 2009, 284, 16998), AZD2836 (AztraZeneca company public website: http://www.astrazeneca-annualreports.com/2007/our performance/therapy area review/infection medicines/ pipeline.asp), BMS-824393 (http://clinicaltrialsfeeds.org/clinical-trials/show/NCT00971308), PF-04878691 (http://clinicaltrials.gov/ct2/show/NCT00810758), Locteron (De Leede, L.G. ; J Interferon Cytokine Res, 2008, 28, 113*),* Omega interferon (Buckwold, V. E.; Antiviral Res, 2007, 73, 118), PEG-Interferon lambda (Marcello, T.; Gastroenterology, 2006, 131, 1887), taribavirin (Gish, R.G., et al., J Hepatol., 2007, 47, 51), VX-950/telapravir (Sarrazin, C., et al., Gastroenterology, 2007, 132, 1767), SCH-503034/boceprevir (Njoroge, F. G., et al., Acc. Chem. Res., 2008, 41, 50), Interferon α-2a, and IMO-2125 (TLR9) (http://clinicatrials.gov/ct2/show/NCT00728936).

### METHODS OF TREATMENT AND PREVENTION OF HEPATITIS C VIRAL INFECTIONS

Disclosed are compositions and combinations for use in treating or preventing a hepatitis C virus infection in a patient in need thereof.

Disclosed are also compositions and combinations for use in introducing a therapeutically effective amount of the combination of compounds into the blood stream of a patient in the treatment and/or prevention of hepatitis C viral infections.

The magnitude of a prophylactic or therapeutic dose of a composition of the invention or a pharmaceutically acceptable salt, solvate, or hydrate, thereof in the acute or chronic treatment or prevention of an infection will vary, however, with the nature and severity of the infection, and the route by which the active ingredient is administered. The dose, and in some cases the dose frequency, will also vary according to the infection to be treated, the age, body weight, and response of the individual patient. Suitable dosing regimens can be readily selected by those skilled in the art with due consideration of such factors.

The uses of the present invention are particularly well suited for human patients. In particular, the uses and doses of the present invention can be useful for immunocompromised patients including, but not limited to cancer patients, HIV infected patients, and patients with an immunodegenerative disease. Furthermore, the methods can be useful for immunocompromised patients currently in a state of remission. The uses and doses of the present invention are also useful for patients undergoing other antiviral treatments. The prevention uses of the present invention are particularly useful for patients at risk of viral infection. These patients include, but are not limited to health care workers, e.g., doctors, nurses, hospice care givers; military personnel; teachers; childcare workers; patients traveling to, or living in, foreign locales, in particular third world locales including social aid workers, missionaries, and foreign diplomats. Finally, the methods and compositions include the treatment of refractory patients or patients resistant to treatment such as resistance to polymerase inhibitors, protease inhibitors, etc.

### Doses

Toxicity and efficacy of the compounds used in the context of the invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀.

The data obtained from the cell culture assays and animal studies can be used in formulating a range of dosage of the compounds for use in humans. The dosage of such compounds lies preferably within a range of circulating concentrations that include the ED₅₀ with little or no toxicity. Preferably, the dosage is in a range that includes the ED₉₅ with manageable toxicity, more preferably with little or no toxicity. The dosage may vary within these ranges depending upon the dosage form employed and the route of administration utilized. For any compound used in context of the invention, the therapeutically effective dose can be estimated initially from cell culture assays. A dose may be formulated in animal models to achieve a circulating plasma concentration range that includes the EC₅₀ (i.e., the concentration of the test compound that achieves a half-maximal inhibition of symptoms) as determined in cell culture; alternatively, the dose of the Formula I compound may be formulated in animal models to achieve a circulating plasma concentration range of the compound that corresponds to the concentration required to achieve a fixed magnitude of response. Such information can be used to more accurately determine useful doses in humans. Levels in plasma may be measured, for example, by high performance liquid chromatography.

The protocols and compositions disclosed herein are preferably tested *in vitro,* and then *in vivo,* for the desired therapeutic or prophylactic activity, prior to use in humans. For example, *in vitro* assays which can be used to determine whether administration of a specific therapeutic protocol is indicated, include *in vitro* cell culture assays in which cells that are responsive to the effects of *N-*{3-[{1*R,*2*S,*7*R,*8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide are exposed to the ligand and the magnitude of response is measured by an appropriate technique. The assessment of the combination compositions is then evaluated with respect to the combination potency. Compositions for use according to the invention can be tested in suitable animal model systems prior to testing in humans, including but not limited to in rats, mice, chicken, cows, monkeys, rabbits, hamsters, etc. The compounds can then be used in the appropriate clinical trials.

The magnitude of a prophylactic or therapeutic dose of *N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide or a pharmaceutically acceptable salt, solvate, or hydrate thereof in combination with a second antiviral agent in the acute or chronic treatment or prevention of an infection or condition will vary with the nature and severity of the infection, and the route by which the active ingredient is administered. The dose, and perhaps the dose frequency, will also vary according to the infection to be treated, the age, body weight, and response of the individual patient. Suitable dosing regimens can be readily selected by those skilled in the art with due consideration of such factors. In one embodiment, the dose administered depends upon the specific compound to be used, and the weight and condition of the patient. Also, the dose may differ for various particular second antiviral compounds; suitable doses can be predicted on the basis of the aforementioned *in vitro* measurements and on the basis of animal studies, such that smaller doses will be suitable for those compositions that show effectiveness at lower concentrations than other compositions when measured in the systems described or referenced herein. In general, the dose of *N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide per day is in the range of from about 0.001 to 100 mg/kg, preferably about 1 to 25 mg/kg, more preferably about 2.5 to 15 mg/kg. For treatment of humans infected by hepatitis C viruses, about 0.1 mg to about 15 g per day is administered in about one to four divisions a day, preferably 100 mg to 12 g per day, more preferably from 100 mg to 2000 mg per day.

Additionally, the recommended daily dose of each agent in the composition can be administered in cycles as single agents or in combination with other therapeutic agents. In one embodiment, the daily dose is administered in a single dose or in equally divided doses. In a related embodiment, the recommended daily dose can be administered once time per week, two times per week, three times per week, four times per week or five times per week.

In one embodiment, the compositions of the invention are administered to provide systemic distribution of the compound within the patient. In a related embodiment, the compositions of the invention are administered to produce a systemic effect in the body.

In another embodiment the compositions of the invention are administered via oral, mucosal (including sublingual, buccal, rectal, nasal, or vaginal), parenteral (including subcutaneous, intramuscular, bolus injection, intraarterial, or intravenous), transdermal, or topical administration. In a specific embodiment the compositions of the invention are administered via mucosal (including sublingual, buccal, rectal, nasal, or vaginal), parenteral (including subcutaneous, intramuscular, bolus injection, intraarterial, or intravenous), transdermal, or topical administration. In a further specific embodiment, the compositions of the invention are administered via oral administration. In a further specific embodiment, the compositions of the invention are not administered via oral administration.

Different therapeutically effective amounts may be applicable for different infections, as will be readily known by those of ordinary skill in the art. Similarly, amounts sufficient to treat or prevent such infections, but insufficient to cause, or sufficient to reduce, adverse effects associated with conventional therapies are also encompassed by the above described dosage amounts and dose frequency schedules.

### PHARMACEUTICAL COMPOSITIONS AND DOSAGE FORMS

Pharmaceutical compositions and single unit dosage forms comprising a composition of the invention, or pharmaceutically acceptable salts, or hydrates thereof, are also encompassed by the invention. Individual dosage forms of the invention may be suitable for oral, mucosal (including sublingual, buccal, rectal, nasal, or vaginal), parenteral (including subcutaneous, intramuscular, bolus injection, intraarterial, or intravenous), transdermal, or topical administration. Pharmaceutical compositions and dosage forms of the invention typically also comprise one or more pharmaceutically acceptable excipients. Sterile dosage forms are also contemplated.

In an alternative embodiment, pharmaceutical composition encompassed by this embodiment includes a composition of the invention, or pharmaceutically acceptable salts, or hydrates thereof, and at least one additional therapeutic agent. Examples of additional therapeutic agents include, but are not limited to, those listed above.

The composition, shape, and type of dosage forms will typically vary depending on their use. For example, a dosage form used in the acute treatment of a disease or a related disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease or disorder. These and other ways in which specific dosage forms encompassed by this invention will vary from one another will be readily apparent to those skilled in the art. *See,* e.g., Remington's Pharmaceutical Sciences, 18th ed., Mack Publishing, Easton PA (1990). Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

Typical pharmaceutical compositions and dosage forms comprise one or more carriers, excipients or diluents. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form.

This invention further encompasses anhydrous pharmaceutical compositions and dosage forms comprising active ingredients, since water can facilitate the degradation of some compounds. For example, the addition of water (e.g., 5%) is widely accepted in the pharmaceutical arts as a means of simulating long-term storage in order to determine characteristics such as shelf-life or the stability of formulations over time. See, e.g., Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

The invention further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms of the invention comprise compositions of the invention, or pharmaceutically acceptable salts or hydrates thereof, comprise 0.1 mg to 1500 mg per unit to provide doses of about 0.01 to 200 mg/kg per day.

### Oral Dosage Forms

Pharmaceutical compositions of the invention that are suitable for oral administration can be presented as discrete dosage forms, such as, but are not limited to, tablets (e.g., chewable tablets), caplets, capsules, and liquids (e.g., flavored syrups). Such dosage forms contain predetermined amounts of active ingredients, and may be prepared by methods of pharmacy well known to those skilled in the art. See generally, *Remington's Pharmaceutical Sciences,* 18th ed., Mack Publishing, Easton PA (1990).

Typical oral dosage forms of the invention are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (e.g., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Lubricants that can be used in pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### Delayed Release Dosage Forms

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### Parenteral Dosage Forms

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry and/or lyophylized products ready to be dissolved or suspended in a pharmaceutically acceptable vehicle for injection (reconstitutable powders), suspensions ready for injection, and emulsions.

Suitable vehicles that can be used to provide parenteral dosage forms of the invention are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention.

### Transdermal Dosage Forms

Transdermal dosage forms include "reservoir type" or "matrix type" patches, which can be applied to the skin and worn for a specific period of time to permit the penetration of a desired amount of active ingredients.

Suitable excipients (e.g., carriers and diluents) and other materials that can be used to provide transdermal and topical dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof.

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### Topical Dosage Forms

Topical dosage forms of the invention include, but are not limited to, creams, lotions, ointments, gels, solutions, emulsions, suspensions, or other forms known to one of skill in the art. *See, e.g.,* Remington's Pharmaceutical Sciences, 18th eds., Mack Publishing, Easton PA (1990); and Introduction to Pharmaceutical Dosage Forms, 4th ed., Lea & Febiger, Philadelphia (1985).

Suitable excipients (*e.g.,* carriers and diluents) and other materials that can be used to provide transdermal and topical dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof.

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

### Mucosal Dosage Forms

Mucosal dosage forms of the invention include, but are not limited to, ophthalmic solutions, sprays and aerosols, or other forms known to one of skill in the art. See, *e.g.,* Remington's Pharmaceutical Sciences, 18th eds., Mack Publishing, Easton PA (1990); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. In one embodiment, the aerosol comprises a carrier. In another embodiment, the aerosol is carrier free.

The compositions of the invention may also be administered directly to the lung by inhalation. For administration by inhalation, a composition can be conveniently delivered to the lung by a number of different devices. For example, a Metered Dose Inhaler ("MDI") which utilizes canisters that contain a suitable low boiling propellant, e.g., dichlorodifluoromethane, trichlorofluoromethane, dichlorotetrafluoroethane, carbon dioxide or other suitable gas can be used to deliver a Formula I compound directly to the lung. MDI devices are available from a number of suppliers such as 3M Corporation, Aventis, Boehringer Ingelheim, Forest Laboratories, Glaxo-Wellcome, Schering Plough and Vectura.

Alternatively, a Dry Powder Inhaler (DPI) device can be used to administer a composition of the invention to the lung (*see, e.g.,* Raleigh et al., Proc. Amer. Assoc. Cancer Research Annual Meeting, 1999, 40, 397. DPI devices typically use a mechanism such as a burst of gas to create a cloud of dry powder inside a container, which can then be inhaled by the patient. DPI devices are also well known in the art and can be purchased from a number of vendors which include, for example, Fisons, Glaxo-Wellcome, Inhale Therapeutic Systems, ML Laboratories, Qdose and Vectura. A popular variation is the multiple dose DPI ("MDDPI") system, which allows for the delivery of more than one therapeutic dose. MDDPI devices are available from companies such as AstraZeneca, GlaxoWellcome, IVAX, Schering Plough; SkyePharma and Vectura. For example, capsules and cartridges of gelatin for use in an inhaler or insufflator can be formulated containing a powder mix of the compound and a suitable powder base such as lactose or starch for these systems.

Another type of device that can be used to deliver a composition of the invention to the lung is a liquid spray device supplied, for example, by Aradigm Corporation. Liquid spray systems use extremely small nozzle holes to aerosolize liquid drug formulations that can then be directly inhaled into the lung.

In one embodiment, a nebulizer device is used to deliver a composition of the invention to the lung. Nebulizers create aerosols from liquid drug formulations by using, for example, ultrasonic energy to form fine particles that can be readily inhaled (See e.g., Verschoyle et al., British J. Cancer, 1999, 80, Suppl 2, 96). Examples of nebulizers include devices supplied by Sheffield/Systemic Pulmonary Delivery Ltd. (See, Armer et al., U.S. Pat. No. 5,954,047; van der Linden et al., U.S. Pat. No. 5,950,619; van der Linden et al., U.S. Pat. No. 5,970,974), Aventis and Batelle Pulmonary Therapeutics.

In one embodiment, an electrohydrodynamic ("EHD") aerosol device is used to deliver compositions of the invention to the lung. EHD aerosol devices use electrical energy to aerosolize liquid drug solutions or suspensions (*see, e.g.,* Noakes et al., U.S. Pat. No. 4,765,539; Coffee, U.S. Pat. No., 4,962,885; Coffee, PCT Application, WO 94/12285; Coffee, PCT Application, WO 94/14543; Coffee, PCT Application, WO 95/26234, Coffee, PCT Application, WO 95/26235, Coffee, PCT Application, WO 95/32807). The electrochemical properties of the Formula I compounds formulation may be important parameters to optimize when delivering this drug to the lung with an EHD aerosol device and such optimization is routinely performed by one of skill in the art. EHD aerosol devices may more efficiently delivery drugs to the lung than existing pulmonary delivery technologies. Other methods of intra-pulmonary delivery of Formula I compounds will be known to the skilled artisan.

Liquid drug formulations suitable for use with nebulizers and liquid spray devices and EHD aerosol devices will typically include a composition of the invention with a pharmaceutically acceptable carrier. Preferably, the pharmaceutically acceptable carrier is a liquid such as alcohol, water, polyethylene glycol or a perfluorocarbon. Optionally, another material may be added to alter the aerosol properties of the solution or suspension of the composition of the invention. Preferably, this material is liquid such as an alcohol, glycol, polyglycol or a fatty acid. Other methods of formulating liquid drug solutions or suspension suitable for use in aerosol devices are known to those of skill in the art *(see, e.g.,* Biesalski, U.S. Pat. Nos. 5,112,598; Biesalski, 5,556,611). A composition of the invention can also be formulated in rectal or vaginal compositions such as suppositories or retention enemas, *e.g.,* containing conventional suppository bases such as cocoa butter or other glycerides.

In addition to the formulations described previously, a composition of the invention can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

Alternatively, other pharmaceutical delivery systems can be employed. Liposomes, emulsions, self-emulsifying (SEDDS), and self micro-emulsifying systems (SMEDDS) are well known examples of delivery vehicles that can be used to deliver compositions of the invention. Such systems can also contain fatty acids, bile salts and mixtures of mono-, di- and triglycerides to ameliorate potential food effects. Other functional lipid excipients include esters of glycerol, PEG-esters, propylene glycol esters and polyglycerol esters. Certain organic solvents such as dimethylsulfoxide can also be employed, although usually at the cost of greater toxicity. A composition of the invention can also be delivered in a controlled release system. In one embodiment, a pump can be used (Sefton, CRC Crit. Ref Biomed Eng., 1987, 14,201; Buchwald et al., Surgery, 1980, 88, 507; Saudek et al., N. Engl. J. Med., 1989, 321, 574). In another embodiment, polymeric materials can be used (see Medical Applications of Controlled Release, Langer and Wise (eds.), CRC Pres., Boca Raton, Fla. (1974); Controller Drug Bioavailability, Drug Product Design and Performance, Smolen and Ball (eds.), Wiley, New York (1984); Ranger and Peppas, J. Macromol. Sci. Rev. Macromol. Chem., 1983, 23, 61; see also Levy et al., Science, 1985,228, 190; During et al., Ann. Neurol., 1989,25,351; Howard et al., J. Neurosurg., 71, 105 (1989). In yet another embodiment, a controlled-release system can be placed in proximity of the target of the compounds used in the context of the invention, e.g., the lung, thus requiring only a fraction of the systemic dose (see, *e.g.,* Goodson, in Medical Applications of Controlled Release, supra, vol. 2, pp. 115 (1984)). Other controlled-release system can be used (see, e.g., Langer, Science, 1990, 249, 1527).

Suitable excipients (e.g., carriers and diluents) and other materials that can be used to provide mucosal dosage forms encompassed by this invention are well known to those skilled in the pharmaceutical arts, and depend on the particular site or method which a given pharmaceutical composition or dosage form will be administered. With that fact in mind, typical excipients include, but are not limited to, water, ethanol, ethylene glycol, propylene glycol, butane-1,3-diol, isopropyl myristate, isopropyl palmitate, mineral oil, and mixtures thereof, which are non-toxic and pharmaceutically acceptable. Examples of such additional ingredients are well known in the art. See, e.g., Remington's Pharmaceutical Sciences, 18th eds., Mack Publishing, Easton PA (1990).

The pH of a pharmaceutical composition or dosage form, or of the tissue to which the pharmaceutical composition or dosage form is applied, can also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### KITS

Disclosed is also a pharmaceutical pack or kit comprising one or more containers comprising a composition of the invention useful for the treatment or prevention of a Hepatitis C virus infection. Furthermore disclosed is a pharmaceutical pack or kit comprising one or more containers comprising a Formula I compound useful for the treatment or prevention of a Hepatitis C virus infection and one or more containers comprising an additional therapeutic agent, including but not limited to those listed above, in particular an antiviral agent, an interferon, an agent which inhibits viral enzymes, or an agent which inhibits viral replication, preferably the additional therapeutic agent is HCV specific or demonstrates anti-HCV activity.

Disclosed is furthermore a pharmaceutical pack or kit comprising one or more containers comprising one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The *N*-{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide for combination with one or more additional antiviral compounds may be prepared using the reaction routes and synthesis schemes as described below, employing the general techniques known in the art using starting materials that are readily available. The synthesis of non-exemplified compounds according to the invention may be successfully performed by modifications apparent to those skilled in the art, e.g., by appropriately protecting interfering groups, by changing to other suitable reagents known in the art, or by making routine modifications of reaction conditions. Alternatively, other reactions disclosed herein or generally known in the art will be recognized as having applicability for preparing other compounds of the invention.

### Preparation of Compounds

In the synthetic schemes described below, unless otherwise indicated all temperatures are set forth in degrees Celsius and all parts and percentages are by weight.

Reagents were purchased from commercial suppliers such as Aldrich Chemical Company or Lancaster Synthesis Ltd. and were used without further purification unless otherwise indicated. All solvents were purchased from commercial suppliers such as Aldrich, EMD Chemicals or Fisher and used as received.

The reactions set forth below were done generally under a positive pressure of argon or nitrogen at an ambient temperature (unless otherwise stated) in anhydrous solvents, and the reaction flasks were fitted with rubber septa for the introduction of substrates and reagents via syringe. Glassware was oven dried and/or heat dried.

The reactions were assayed by TLC and/or analyzed by LC-MS or HPLC and terminated as judged by the consumption of starting material. Analytical thin layer chromatography (TLC) was performed on glass-plates precoated with silica gel 60 F₂₅₄ 0.25 mm plates (EMD Chemicals), and visualized with UV light (254 nm) and/or iodine on silica gel and/or heating with TLC stains such as ethanolic phosphomolybdic acid, ninhydrin solution, potassium permanganate solution or ceric sulfate solution. Preparative thin layer chromatography (prepTLC) was performed on glass-plates precoated with silica gel 60 F₂₅₄ 0.5 mm plates (20 × 20 cm, from Thomson Instrument Company) and visualized with UV light (254 nm).

Work-ups were typically done by doubling the reaction volume with the reaction solvent or extraction solvent and then washing with the indicated aqueous solutions using 25% by volume of the extraction volume unless otherwise indicated. Product solutions were dried over anhydrous Na₂SO₄ and/or MgSO₄ prior to filtration and evaporation of the solvents under reduced pressure on a rotary evaporator and noted as solvents removed *in vacuo.* Column chromatography was completed under positive pressure using Merck silica gel 60, 230-400 mesh or 50-200 mesh neutral alumina, ISCO Flash -chromatography using prepacked RediSep silica gel columns, or Analogix flash column chromatography using prepacked SuperFlash silica gel columns. Hydrogenolysis was done at the pressure indicated in the examples or at ambient pressure.

¹H-NMR spectra and ¹³C-NMR were recorded on a Varian Mercury-VX400 instrument operating at 400 MHz. NMR spectra were obtained as CDCl₃ solutions (reported in ppm), using chloroform as the reference standard (7.27 ppm for the proton and 77.00 ppm for carbon), CD₃OD (3.4 and 4.8 ppm for the protons and 49.3 ppm for carbon), DMSO-d₆ (2.49 ppm for proton), or internally tetramethylsilane (0.00 ppm) when appropriate. Other NMR solvents were used as needed. When peak multiplicities are reported, the following abbreviations are used: s (singlet), d (doublet), t (triplet), q (quartet), m (multiplet), br (broadened), bs (broad singlet), dd (doublet of doublets), dt (doublet of triplets). Coupling constants, when given, are reported in Hertz (Hz).

Infrared (IR) spectra were recorded on an ATR FT-IR Spectrometer as neat oils or solids, and when given are reported in wave numbers (cm⁻¹). Mass spectra reported are (+)-ES or APCI (+) LC/MS conducted by the Analytical Chemistry Department of Anadys Pharmaceuticals, Inc. Elemental analyses were conducted by the Atlantic Microlab, Inc. in Norcross, GA. Melting points (mp) were determined on an open capillary apparatus, and are uncorrected.

Enantiomeric excess (ee) values were determined by HPLC-analysis using the Chiralpak (Chiral Technologies Inc.) columns AS-RH, 2.1 × 150 mm, 5 micron, λ = 312 nm or AS-RH, 4.6 × 250 mm, 5 micron, λ = 310 nm.

AS-RH, 2.1 × 150 mm, 5 micron: Binary gradient HPLC separation. Solvent A: 0.1% Formic Acid in Water, Solvent B: 0.1% Formic Acid in Acetonitrile. Injected 10 µL of sample dissolved in 50% methanol - 50% water [0.1 mg/mL].

| Time (min) | %B | Flow (mL/min) |
|---|---|---|
| 0.0 | 55 | 0.3 |
| 5.0 | 95 | 0.3 |
| 5.5 | 95 | 0.3 |
| 6.0 | 55 | 0.3 |
| 12.0 | 55 | 0.3 |

AS-RH, 4.6 × 250 mm, 5 micron: Binary gradient HPLC separation. Solvent A: 0.05 % TFA in Water, Solvent B: 0.05 % TFA in Acetonitrile. Injected 3-5 µl of sample dissolved in acetonitrile [1 mg/mL].

| Time (min) | %B | Flow (mL/min) |
|---|---|---|
| 0.0 | 50 | 0.8 |
| 8.0 | 95 | 0.8 |
| 10.0 | 95 | 0.8 |
| 11.0 | 50 | 0.8 |
| 13.0 | 50 | 0.8 |

The described synthetic pathways and experimental procedures utilize many common chemical abbreviations, 2,2-DMP (2,2-dimethoxypropane), Ac (acetyl), ACN (acetonitrile), Bn (benzyl), BnOH (benzyl alcohol), Boc (tert-butoxycarbonyl), Boc₂O (di-tert-butyl dicarbonate), Bz (benzoyl), CSI (chlorosulfonyl isocyanate), DBU (1,8-diazabicyclo[5.4.0]undec-7-ene), DCC (*N,N'-*dicyclohexylcarbodiimide), DCE (1,2-dichloroethane), DCM (dichloromethane), DEAD (diethylazodicarboxylate), DIEA (diisopropylethylamine), DMA (*N,N-*dimethylacetamide), DMAP (4-(*N,N*-dimethylamino)pyridine), DMF (*N,N-*dimethylformamide), DMSO (dimethyl sulfoxide), EDC (1-(3-dimethylaminopropyl)-3-ethylcarbodiimide hydrochloride), Et (ethyl), EtOAc (ethyl acetate), EtOH (ethanol), HATU (O-(7-azabenzotriazol-1-yl)-1,1,3,3-tetramethyluronium hexafluorophosphate), HBTU (*O*-benzotriazol-1-yl-*N,N,N,N*'-tetramethyluronium hexafluorophosphate), HF (hydrogen fluoride), HOAc (acetic acid), HOBT (1-hydroxybenzotriazole hydrate), HPLC (high pressure liquid chromatography), IPA (isopropyl alcohol), KHMDS (potassium bis(trimethylsilyl)amide), KN(TMS)₂ (potassium bis(trimethylsilyl)amide), KO*^{t}*Bu (potassium *tert*-butoxide), LDA (lithium diisopropylamine), MCPBA (3-chloroperbenzoic acid), Me (methyl), MeCN (acetonitrile), MeOH (methanol), NaBH(OAc)₃ (sodium triacetoxyborohydride), NaCNBH₃ (sodium cyanoborohydride), NaH (sodium hydride), NaN(TMS)₂ (sodium bis(trimethylsilyl)amide), NaOAc (sodium acetate), NaOEt (sodium ethoxide), Phe (phenylalanine), PPTS (pyridinium p-toluenesulfonate), PS (polymer supported), Py (pyridine), pyBOP (benzotriazol-1-yloxy)tripyrrolidinophosphonium hexafluorophosphate), TEA (triethylamine), TFA (trifluoroacetic acid), TFAA (trifluoroacetic anhydride), THF (tetrahydrofuran), TLC (thin layer chromatography), Tol (toluoyl), Val (valine), and the like.

The preparation of *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide and its salts is disclosed in US Application No. 12/061,499.

### Example 1: (7-Methanesulfonylamino-1,1-dioxo-1,4-dihydro-1λ⁶-benzo[1,2,4]thiadiazin-3-yl)-acetic acid

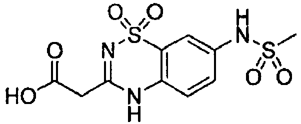

### a) 2-Chloro-5-nitrobenzenesulfonamide

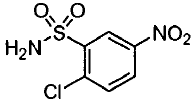

To a solution of thionyl chloride (11 mL) and 2-chloro-5-nitrobenzenesulfonic acid (4.78 g, 20.1 mmol) was added *N,N-*dimethylformamide (0.92 µL) and the reaction mixture was heated at reflux for 4 h. The reaction mixture was then carefully quenched by pouring it into water and the product was isolated by vacuum filtration. The sulfonyl chloride was dissolved in a minimal amount of toluene and then added to a mixture of concentrated aqueous ammonium hydroxide solution (25 mL) and tetrahydrofuran (25 mL) at -10 °C. After stirring for 2 h the reaction was quenched by adding a 6.0 M aqueous hydrochloric acid solution until pH 4 was reached. The layers were separated and the organic layer was concentrated *in vacuo* to a slurry. Pentane was added and the product was isolated by vacuum filtration to afford 2-chloro-5-nitrobenzenesulfonamide (2.0 g, 8.48 mmol, 42.4%), as a solid. ¹H NMR (400 MHz, DMSO-*d₆*) δ: 7.94 (d, 1H, *J=* 8.8 Hz), 7.97 (bs, 2H), 8.40 (dd, 1H, *J₁* = 8.6 Hz*, J₂* = 3.1 Hz), 8.64 (d, 1H, *J* = 3.1 Hz).

### b) 2-Amino-5-nitrobenzenesulfonamide

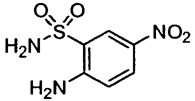

2-Chloro-5-nitro-benzenesulfonamide (1.95 kg, 8.30 mol), ammonium carbonate (1.983 kg, 20.64 mol), and copper (II) sulfate (394 g, 2.47 mol) were charged to an autoclave and diluted with a 30% aqueous ammonium hydroxide solution (11.7 L, 330 mol). The mixture was heated at 118 °C for 3 days and was then cooled to 23 °C. The mixture was filtered and the solids were then washed with water (20 L). This solid was dissolved in hot methanol (20 mL/g), and the mixture was filtered to remove undissolved solids. The filtrate was stored at 4 °C overnight, and the resulting solid product was then filtered. The filtrate was partially concentrated by vacuum distillation and, when the concentrate was cooled to 23 °C, the solid product was then filtered off. The two crops of solid were combined and further dried *in vacuo* at 45 °C to afford the desired product, 2-amino-5-nitro-benzenesulfonamide (1.10 kg, 5.06 mol, 61%), as a solid. ¹H NMR (400 MHz, DMSO-*d₆*) δ: 6.89 (d, *J* = 9.3 Hz, 1H), 7.12 (bs, 2H), 7.57 (bs, 2H), 8.07 (dd, *J₁* = 9.0 Hz, *J₂* = 2.6 Hz, 1H), 8.43 (d, *J=* 3.0 Hz, 1H).

Alternatively, 2-amino-5-nitrobenzenesulfonamide can be prepared as follows:

2-Amino-5-nitrobenzenesulfonic acid (200.00 g, 0.917 mol) was suspended in warm sulfolane (250 mL) and the suspension was heated to 80 °C. Phosphorous oxychloride (126 mL, 1.375 mol) was added and resulting mixture was heated to 110-120 °C and stirred for 4 h. The resulting solution was cooled to 60 °C and added dropwise into concentrated aqueous ammonium hydroxide solution (800 mL, 11.9 mol) at <10 °C. The flask was rinsed with warm sulfolane (50 mL) and the wash was added into the above reaction mixture. The resulting suspension was stirred at 25 °C for 1 h, heated to 95 °C and stirred for 1 hour. The mixture was cooled to 80 °C and the pH was adjusted to 6-8 with 3.0 M aqueous hydrochloric acid solution (∼600 mL) and allowed to cool to 25 °C. The dark green suspension was filtered, and the wet filter cake was washed with water (300 mL) and dried at 60 °C overnight to give the crude product (140 g) as a green-yellow solid. The crude product was dissolved in 0.5 M aqueous sodium hydroxide solution (1.4 L, 0.7 mol). Charcoal (14 g) was added and the mixture was heated to reflux and stirred for 15 min. The mixture was filtered through Celite and washed with 0.5 M aqueous sodium hydroxide solution (100 mL). The pH of the filtrate was adjusted to 6-8 with concentrated aqueous hydrochloric acid solution (∼ 60 mL) and the yellow suspension was allowed to cool to 25 °C. The mixture was filtered and the wet filter cake was washed with water (200 mL) and dried at 60 °C overnight to afford the desired product, 2-amino-5-nitrobenzenesulfonamide (130 g, 0.599 mol, 65%) as a bright yellow powder.

### c) 2,5-Diaminobenzenesulfonamide

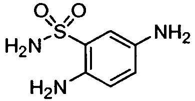

2-Amino-5-nitro-benzenesulfonamide (5.00 kg, 23.0 mol), methanol (65 L), tetrahydrofuran (65 L), and 10% palladium on carbon (250 g) were charged to an autoclave. The mixture was cycled with nitrogen and hydrogen purges (3 ×), and the mixture was then stirred under hydrogen (50 psi) at 23 °C overnight. The catalyst was removed by filtration and the filtrate was then concentrated *in vacuo* to give a brown solid. The solid was further dried *in vacuo* at 45 °C to afford the desired product, 2,5-diamino-benzenesulfonamide (4.21 kg, 22.4 mol, 98%), as a solid. ¹H NMR (400 MHz, DMSO-*d₆*) δ: 4.54 (2H, bs), 4.98 (2H, bs), 6.55 - 6.60 (2H, m), 6.87 (1H, d, *J=* 2.2 Hz), 6.99 (2H, bs). LC-MS (ESI) calcd for C₆H₉N₃O₂S 187.04, found 188.3 [M+H⁺].

### d) 2-Amino-5-methanesulfonylamino-benzenesulfonamide

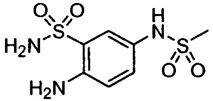

2,5-Diamino-benzenesulfonamide (4.20 kg, 22.4 mol) was dissolved in dichloromethane (120 L) and pyridine (8.00 kg, 89.9 mol), and the resulting solution was cooled to 0 °C. Methanesulfonyl chloride (2.80 kg, 24.4 mol) was added slowly, and the resulting mixture was allowed to warm to 23 °C and stirred for 2 days. The mixture was filtered and the resulting solid was washed with dichloromethane (2 × 20 L). The solid was diluted with water (100 L) and 1.0 M aqueous hydrochloric acid solution (25 L), and was then stirred at 23 °C for 1 h. The mixture was filtered and the resulting solid was washed with water (20 L) and then with methyl-*tert*-butyl ether (2 × 10 L). The solid was further dried *in vacuo* at 45 °C to afford the desired product, 2-amino-5-methanesulfonylamino-benzenesulfonamide (4.39 kg, 16.5 mol, 73%) as a pale pink solid. ¹H NMR (400 MHz, CD₃OD) δ: 2.89 (3H, s), 6.82 (1H, d, *J=* 8.5 Hz), 7.20 (1H, dd, *J₁* = 8.5 Hz, *J₂* = 2.5 Hz), 7.58 (1H, d, *J=* 2.5 Hz). LC-MS (ESI) calcd for C₇H₁₁N₃O₄S₂ 265.02, found 266.0 [M+H⁺].

Alternatively, 2-amino-5-methanesulfonylamino-benzenesulfonamide can be prepared as follows:
a') 2-Benzylamino-5-nitro-benzenesulfonamide

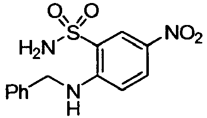

A mixture of 2-chloro-5-nitro-benzenesulfonamide (2.20 kg, 9.30 mol), benzylamine (1.5 L, 13.9 mol), triethylamine (2.5 L, 18.1 mol), and acetonitrile (22.0 L) were heated at 92 °C for 20 h. The mixture was then cooled to 40 °C, and was then partially concentrated *in vacuo.* The residue was added to 0 °C water (22.0 L) and the resulting suspension was allowed to warm to 23 °C and stirred for 2 h. The suspension was filtered and the solid was then washed with water (5 L). The washed solid was suspended in absolute ethanol (11 L), and was then filtered and washed with absolute ethanol (5 L). The solid was further dried *in vacuo* at 45 °C to afford the desired product, 2-benzylamino-5-nitro-benzenesulfonamide (2.40 kg, 7.81 mol, 84%), as a yellow solid. ¹H NMR (400 MHz, DMSO-*d₆*) δ: 4.64 (2H, d, *J*= 4.6 Hz), 6.81 (1H, d, *J =* 9.4 Hz), 7.23 - 7.44 (6H, m), 7.77 (2H, bs), 8.11 (1H, dd, *J₁* = 9.4 Hz, *J*₂ = 2.3 Hz), 8.49 (1H, d, *J=* 3.1 Hz). LC-MS (ESI) calcd for C₁₃H₁₃N₃O₄S307.06, found 308.2 [M+H⁺] (100%), 615.2 [2M+H⁺] (81%).

### b') 2,5-Diamino-benzenesulfonamide methanesulfonate

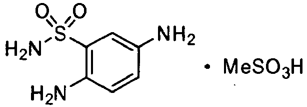

Methanesulfonic acid (465 mL, 7.16 mol) was added slowly to a solution of 2-benzylamino-5-nitro-benzenesulfonamide (2.20 kg, 7.16 mol) and tetrahydrofuran (11.0 L). The resulting solution was added to a mixture of 10% palladium on carbon (220 g of 50% water wet catalyst) and water (1.1 L) in a hydrogenation reactor. The mixture was further diluted with absolute ethanol (21.0 L) and was then hydrogenated with 55 psi hydrogen at 50 °C for 21 h. Additional 10% palladium on carbon (55 g of 50% water wet catalyst) was added, and hydrogenation at 55 psi and 50 °C was continued for 22 h. The resulting suspension was diluted with water (1.1 L) and the suspension was then filtered through a pad of Celite. The filtrate was partially concentrated *in vacuo* and was then diluted with acetonitrile (15.4 L). The solution was again partially concentrated *in vacuo* and diluted with acetonitrile (15.4 L). The resulting suspension was partially concentrated *in vacuo* and was allowed to stir at 23 °C for 2 h. The suspension was filtered and the solid was then washed with acetonitrile (3 L). The solid was further dried *in vacuo* at 45 °C to afford the desired product, 2,5-diamino-benzenesulfonamide methanesulfonate (1.88 kg, 6.64 mol, 93%), as a purple solid. ¹H NMR (400 MHz, DMSO-*d₆*) δ: 2.34 (3H, s), 6.05 (2H, b), 6.87 (1H, d, *J=* 8.6 Hz), 7.20 (1H, dd, *J₁* = 8.6 Hz, *J₂* = 2.3 Hz), 7.38 (2H, s), 7.53 (1H, d, *J=* 2.3 Hz), 9.62 (3H, b). LC-MS (ESI) calcd for C₆H₉NO₂S 187.04, found 187.9 [M+H⁺].

Alternatively, 2,5-diamino-benzenesulfonamide methanesulfonate can be prepared as follows:

2-Amino-5-nitrobenzenesulfonamide (prepared as described in Example 1b, 100.00 g, 0.460 mol) and 5 % palladium on carbon (wet, 5.00 g) were suspended in ethanol (2 L) and water (100 mL). Methanesulfonic acid (33 mL, 0.51 mol) was added, and the resulting mixture was heated to 55 °C and stirred under atmospheric hydrogen for 8 h. The mixture was filtered and the filtrate was concentrated *in vacuo* to a volume of about 450 mL. To the concentrate was added acetonitrile (1 L) and resulting mixture was stirred at 25 °C overnight. The suspension was filtered to afford the desired product, 2,5-diamino-benzenesulfonamide methanesulfonate (122.36 g, 0.432 mol, 93.8%) as a purple solid.

### c') 2-Amino-5-methanesulfonylamino-benzenesulfonamide

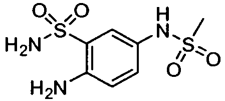

2,5-Diamino-benzenesulfonamide methanesulfonate (1.80 kg, 6.35 mol) was suspended in acetonitrile (24 L). Pyridine (1.55 L, 19.1 mol) was added, followed by the careful slow addition of methanesulfonyl chloride (517 mL, 6.68 mol). After stirring at 23 °C for 20 h, the mixture was partially concentrated *in vacuo* at 55 °C. Water (18 L) was added to the concentrate, and the resulting suspension was stirred at 23 °C for 2 h. The solid was filtered and was then washed with water (4 L) and air dried on the filter. The solid was suspended in absolute ethanol (9 L), stirred at 23 °C for 9 h, and was then filtered. The solid was washed with absolute ethanol (2 × 2 L), and was then further dried *in vacuo* at 50 °C to afford the desired product, 2-amino-5-methanesulfonylamino-benzenesulfonamide (1.45 kg, 5.48 mol, 96%), as a purple solid.

### e) N-(4-Methanesulfonylamino-2-sulfamoyl-phenyl)-malonamic acid ethyl ester

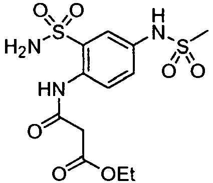

2-Amino-5-methanesulfonylamino-benzenesulfonamide (23.27 g, 87.81 mmol) was dissolved in *N,N-*dimethylacetamide (100 mL) and diethyl ether (100 mL). Ethyl 3-chloro-3-oxo-propionate (13.88 g, 92.20 mmol) was added and the reaction mixture was stirred at 25 °C for 1 h. The reaction mixture was diluted with ethyl acetate (400 mL) and was extracted with water (400 mL). The aqueous layer was back-extracted with ethyl acetate (2 × 200 mL). The combined organic layers were dried over sodium sulfate, filtered, and most of the solvent was removed in *vacuo* to a volume of ∼100 mL.

To the stirred solution was added hexanes (∼100 mL) upon which a precipitate formed. The precipitate was collected by vacuum filtration, washed with hexanes and dried under high vacuum to afford the analytically pure product, *N*-(4-methanesulfonylamino-2-sulfamoyl-phenyl)-malonamic acid ethyl ester (31.22 g, 85.53 mmol, 97.4%), as a light-brown solid. ¹H NMR (400 MHz, CD₃OD) δ: 1.31 (3H, t, *J=* 7.0 Hz), 3.00 (3H, s), 3.59 (2H, s), 4.25 (2H, quartet, *J=* 6.9 Hz), 7.42-7.45 (1H, m), 7.86 (1H, m), 7.92 (1H, d, *J=* 8.8 Hz).

### f) N-(4-Methanesulfonylamino-2-sulfamoylphenyl)-malonamic acid methyl ester

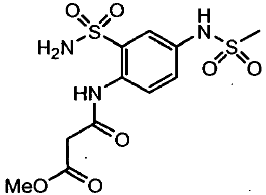

2-Amino-5-methanesulfonylamino-benzenesulfonamide (prepared as described in Example 1d, 1.70 kg, 6.40 mol) was dissolved in tetrahydrofuran (35 L), and was then cooled to 0 °C. Methyl 3-chloro-3-oxopropionate (792 mL, 7.40 mol) was added slowly, and the resulting mixture was then allowed to warm to 23 °C and stirred for 2 days. The solvent was removed *in vacuo,* and the residue was then diluted with water (4 L) and saturated aqueous sodium bicarbonate solution (2 L). The resulting solid was filtered, and was then washed with water (5 L). The solid was suspended in hot methanol (15 mL/g), and was then cooled to 23 °C and filtered to afford the desired product, N-(4-methanesulfonylamino-2-sulfamoyl-phenyl)-malonamic acid methyl ester (1:68 kg, 4.61 mol, 72%), as a brown solid. ¹H NMR (400 MHz, DMSO-*d*₆) δ: 3.02 (3H, s), 3.60 (2H, s), 3.66 (3H, s), 7.38 (1H, dd, *J₁ =* 2.3 Hz, *J₂* = 8.6 Hz), 7.53 (2H, bs), 7.73 (1H, d, *J=* 2.4 Hz), 7.83 (1H, d, *J=* 8.7 Hz), 9.43 (1H, s), 9.99 (1H, s).

### g) (7-Methanesulfonylamino-1,1-dioxo-1,4-dihydro-1λ⁶-benzo[1,2,4]thiadiazin-3-yl)-acetic acid

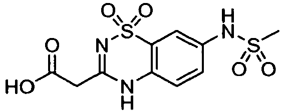

*N*-(4-Methanesulfonylamino-2-sulfamoyl-phenyl)-malonamic acid ethyl ester (prepared as described in Example 1e, 9.55 g, 26.16 mmol) was dissolved in 8% aqueous sodium hydroxide solution (262 mL) and heated at 100 °C for 1.5 h. The reaction mixture was cooled to 0 °C and the solution was acidified by slowly adding 12.0 M aqueous hydrochloric acid solution until pH 1-2 was reached. A precipitate started to form and the suspension was allowed to stir for 30 min at 0 °C. The precipitate was collected by vacuum filtration, washed with cold water, and dried under high vacuum to afford the desired product, (7-methanesulfonylamino-1,1-dioxo-1,4-dihydro-1λ⁶-benzo[1,2,4]thiadiazin-3-yl)-acetic acid (7.20 g, 21.621 mmol, 82.6%), as a pinkish solid. ¹H NMR (400 MHz, DMSO-*d₆*) δ: 3.03 (3H, s), 3.56 (2H, s), 7.33 (1H, d, *J* = 9.1 Hz), 7.52 - 7.54 (2H, m), 10.09 (1H, s), 12.24 (1H, s), 13.02 (1H, bs). LC-MS (ESI) calcd for C₁₀H₁₁N₃O₆S₂ 333.01, found 334.1 [M+H⁺].

Alternatively, (7-methanesulfonylamino-1,1-dioxo-1,4-dihydro-1λ⁶-benzo[1,2,4]thiadiazin-3-yl)-acetic acid can be prepared as follows:

*N*-(4-methanesulfonylamino-2-sulfamoyl-phenyl)-malonamic acid methyl ester (prepared as described in Example 1g, 1.35 kg, 3.69 mol) was added to 3.8 wt. % aqueous sodium hydroxide solution (14.0 kg). The resulting mixture was stirred at 23 °C for 30 h, and was then cooled to 0 °C. A 2.0 M aqueous hydrochloric acid solution (9.72 L) was slowly added, stirring at 0 °C was continued for 30 min, and the mixture was then filtered. The solid was washed with water (1.4 L), and was then slurried in a mixture of methanol (1.4 L) and diethyl ether (2.7 L). After filtration, the solid was washed with diethyl ether (2 × 1.4 L) and was further dried *in vacuo* at 23 °C to afford the desired product, (7-methanesulfonylamino-1,1-dioxo-1,4-dihydro-1λ⁶-benzo[1,2,4]thiadiazin-3-yl)-acetic acid (1.07 kg, 3.21 mol, 87%), as a light brown solid.

### Example 2: N-{3-[(1R,2S,7R,8S)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide

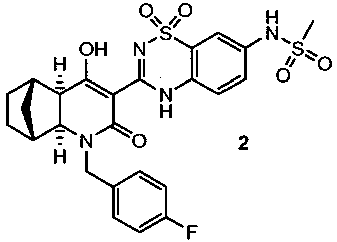

### a) (1S,2S,3R,4R)-3-(Methoxycarbonyl)bicyclo[2.2.1]hept-5-ene-2-carboxylic acid

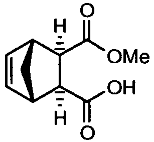

The starting material (a) was prepared as described in J. Org. Chem. 2000, 65, 6984-6991. *cis*-5-Norbornene-exo-2,3-dicarboxylic anhydride (5 g, 30.45 mmol) was suspended in a 1:1 mixture of toluene and carbon tetrachloride (610 mL). The mixture was stirred for 10 min. Quinine (10.87 g, 33.5 mmol) was added and the flask was degassed and backfilled with nitrogen. The solution was cooled to -55 °C. While stirring, methanol (3.7 mL, 91.35 mmol) was added. The mixture was stirred at -55 °C. for 16 h. Upon warming to 25 °C, the mixture was concentrated *in vacuo* to a foam. The foam was dissolved in a mixture of ethyl acetate (400 mL) and 1.0 M aqueous hydrochloric acid solution (400 mL). The layers were separated and the organic layer was further washed with 1.0 M aqueous hydrochloric acid solution (2 × 200 mL), saturated aqueous brine solution (100 mL) and dried over magnesium sulfate, filtered, and concentrated *in vacuo* to afford the desired product, (1*S*,2*S*,3*R*,4*R*)-3-(methoxycarbonyl)bicyclo[2.2.1]hept-5-ene-2-carboxylic acid (5.95 g, 30.3 mmol, 99%), as a clear oil. ¹H NMR (400 MHz, DMSO-*d₆*) δ: 1.31 (1H, d, *J* = 8.5 Hz), 1.98 (1H, d, *J=* 8.6 Hz), 2.51 (2H, d, *J=* 1.6 Hz), 2.95 (2H, bs), 3.52 (3H, s), 6.17 - 6.21 (2H, m), 12.16 (1H, s).

### b) Methyl (1R,2R,3S,4S)-3-{[(benzyloxy)carbonyl]amino}bicyclo[2.2.1]hep-5-ene-2-carboxylate

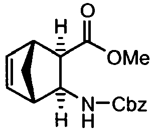

(1*S*,2*S*,3*R*,4*R*)-3-(Methoxycarbonyl)bicyclo[2.2.1]hept-5-ene-2-carboxylic acid (5.9 g, 30 mmol) was dissolved in anhydrous tetrahydrofuran (133 mL). The flask was degassed and backfilled with nitrogen and the mixture was cooled to 0 °C. Triethylamine (12.64 mL, 90 mmol) was added followed by the dropwise addition of ethyl chloroformate (5.72 mL, 60 mmol) with vigorous stirring. Immediate precipitation was observed. The mixture was stirred at 0 °C for 1 h. Sodium azide (5.86 g, 90 mmol) was dissolved in water (40 mL) and added to the reaction mixture at 0 °C. The mixture was stirred at 0 °C for 5 min. The ice bath was removed. The mixture was warmed to 25 °C and continued to stir for 2 h. The mixture was poured into water (300 mL) and the product extracted into ethyl acetate (300 mL). The organic layer was further washed with half-saturated aqueous sodium bicarbonate solution (2 × 100 mL), saturated aqueous brine solution (100 mL), dried over magnesium sulfate, filtered, and concentrated *in vacuo* to afford a light brown oil. The oil was dissolved in anhydrous benzene (66 mL) and refluxed while stirring under nitrogen for 2 h. Upon cooling to 25 °C the solution was concentrated *in vacuo* to afford a light brown oil. The oil was dissolved in dichloromethane (40 mL) and benzyl alcohol (3.41 mL, 33 mmol) was added followed by triethylamine (8.44 mL, 60 mmol). The mixture was refluxed under nitrogen for 16 h. Upon cooling to 25 °C the solution was concentrated *in vacuo* to afford a thick oil. Purification by flash column chromatography (Merck silica gel 60, 40-63 µm; 1^{st} column: 3:1 hexanes/ethyl acetate; 2^{nd} column: 2:4:1 dichloromethane/pentane/diethyl ether) afforded the desired product, methyl (1*R*,2*R*,3*S*,4*S*)-3-{[(benzyloxy)carbonyl]amino}bicyclo[2.2.1]hept-5-ene-2-carboxylate (6.95 g, 23.09 mmol, 77%), as a pale yellow oil. ¹H NMR (400 MHz, CDCl₃) δ: 1.59 (1H, *d, J=* 9.3 Hz), 1.96 (1H, d, *J=* 9.3 Hz), 2.66 (1H, d, *J=* 7.9 Hz), 2.75 (1H, s), 2.96 (1H, s), 3.59 (3H, s), 4.01 (1H, t, *J=* 8.5 Hz), 5.09 (2H, q, *J* = 10.4 Hz), 5.46 (1H, d, *J=* 9.4 Hz), 6.17 - 6.22 (2H, m), 7.29 - 7.36 (5H, m). LC-MS (ESI) calcd for C₁₇H₁₉NO₄ 301.13, found 258.1 (100%), 302.2 [M+H⁺] (70%), 603.5 [2M+H⁺] (20%).

### c) Methyl (1S,2R,3S,4R)-3-aminobicyclo[2.2.1]heptane-2-carboxylate hydrochloride

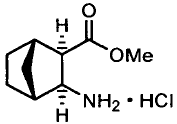

Methyl (1*R*,2*R*,3*S*,4*S*)-3-{[(benzyloxy)carbonyl]amino}bicyclo[2.2.1]hept-5-ene-2-carboxylate (1 g, 3.32 mmol) was dissolved in ethyl acetate (15 mL). 5% Palladium on carbon (120 mg) was added. The flask was degassed and backfilled with hydrogen gas via balloon. The mixture was stirred at 25 °C for 16 h. The mixture was passed through a plug of Celite and the filtrate was concentrated *in vacuo* to afford a thick clear oil. The oil was dissolved in diethyl ether (10 mL) and added dropwise, with vigorous stirring, to a mixture of 4.0 M hydrochloric acid solution in 1,4-dioxane (1.8 mL) in diethyl ether (18 mL). The desired product began to precipitate as a white solid. Additional diethyl ether (10 mL) was added and the mixture was stirred for 10 min. The precipitate was collected by vacuum filtration, washed with additional diethyl ether (2 × 8 mL). The solid was further dried *in vacuo* for 1 h to afford the desired product, methyl (1*S*,2*R*,3*S*,4*R*)-3-aminobicyclo[2.2.1]heptane-2-carboxylate hydrochloride (0.64 g, 3.11 mmol, 94%), as a white powder. ¹H NMR (400 MHz, DMSO-*d₆*) δ: 1.17 - 1.27 (3H, m), 1.40 - 1.61 (2H, m), 1.91 (1H, d, *J* = 10.7 Hz), 2.36 (1H, d, *J* = 4.1 Hz), 2.44 (1H, d, *J* = 3.1 Hz), 2.75 (1H, d, *J* = 7.8 Hz), 3.3 0 - 3.38 (1H, m), 3.61 (3H, s), 8.05 (3H, bs). LC-MS (ESI) calcd for C₉H₁₅NO₂ (free amine) 169.11, found 170.3 [M+H⁺] (100%), 339.3 [2M+H⁺] (50%).

### d) Methyl (1S,2R,3S,4R)-3-[(4-fluorobenzyl)amino]bicyclo[2.2.1]heptane-2-carboxylate

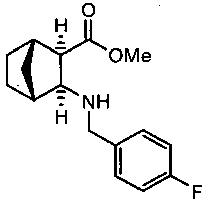

Methyl (1*S*,2*R*,3*S*,4*R*)-3-aminobicyclo[2.2.1]heptane-2-carboxylate hydrochloride (prepared as described in Example 2c, 0.5 g, 2.43 mmol) was dissolved in methanol (12 mL). Sodium acetate (0.4 g, 4.86 mmol) was added followed by 4Å powdered molecular sieves (0.5 g) and 4-fluoro-benzaldehyde (0.302 g, 2.43 mmol). Sodium cyanoborohydride (0.305 g, 4.86 mmol) was added and the mixture was stirred at 25 °C for 16 h. The mixture was poured into a mixture of saturated aqueous sodium bicarbonate solution (200 mL) and ethyl acetate (300 mL). After shaking, both layers were passed through a plug of Celite. The organic layer was further washed with saturated aqueous sodium bicarbonate solution (100 mL), saturated aqueous brine solution (100 mL), dried over magnesium sulfate, filtered, and concentrated *in vacuo* to afford the crude product, methyl (1*S*,2*R*,3*S*,4*R*)-3-[(4-fluorobenzyl)amino]bicyclo[2.2.1]heptane-2-carboxylate (0.663 g, 2.39 mmol, 98%), as a clear oil. LC-MS (ESI) calcd for C₁₆H₂₀FNO₂ 277.15, found 278.2 [M+H⁺].

### e) N-{3-[(1R,2S,7R,8S)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide

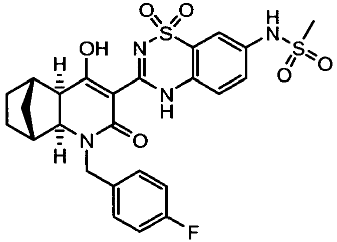

Methyl (1*S*,2*R*,3*S*,4*R*)-3-[(4-fluorobenzyl)amino]bicyclo[2.2.1]heptane-2-carboxylate (0.6 g, 2.16 mmol) was dissolved in anhydrous *N,N-*dimethylformamide (20 mL). (7-Methanesulfonylamino-1,1-dioxo-1,4-dihydro-1λ⁶-benzo[1,2,4]thiadiazin-3-yl)-acetic acid (prepared as described in Example 1, 0.72 g, 2.16 mmol) was added followed by *N-*methylmorpholine (0.5 mL, 4.54 mmol). The mixture was stirred until everything dissolved, approximately 5 min. 1-(3-Dimethylaminopropyl)-3-ethylcarbodiimide hydrochloride (0.435 g, 2.27 mmol) was added and the mixture was stirred at 25 °C for 45 min. Triethylamine (0.91 mL, 6.48 mmol) was added and the mixture was stirred at 50 °C for 16 h.

Upon cooling to 25 °C, the solution was diluted with ethyl acetate (300 mL) and washed with 1.0 M aqueous hydrochloric acid solution (3 × 300 mL), saturated aqueous brine solution (100 mL), dried over magnesium sulfate, filtered, and concentrated *in vacuo* to afford a golden oil. Purification by flash column chromatography (Merck silica gel 60, 40-63 µm, 0 to 0.75% methanol in dichloromethane) afforded the product as white foam. The foam was dissolved in methanol (10 mL) and the product was precipitated by the addition of a 1.0 M aqueous hydrochloric acid solution (20 mL) while stirring. The solid was collected by vacuum filtration and further dried *in vacuo* to afford the desired product, *N-*{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (0.573 g, 1.02 mmol, 47%), as a white powder. ¹H NMR (400 MHz, DMSO-*d₆*) δ: 1.16 - 1.22 (2H, m), 1.37 - 1.65 (4H, m), 2.49 - 2.53 (1H, m), 2.63 (1H, d, *J=* 2.3 Hz), 3.02 (1H, d, *J* = 8.5 Hz), 3.05 (3H, s), 3.52 (1H, d, *J* = 9.4 Hz), 4.41 (1H, d, *J* = 15.6 Hz), 4.95 (1H, d, *J=* 15.6 Hz), 7.14 (2H, t, *J* = 9.0 Hz), 7.32 (2H, dd, *J₁* = 8.1 Hz, *J₂* = 5.7 Hz), 7.50 (1H, dd, *J₁* = 9.5 Hz, *J₂* = 2.3 Hz), 7.55-7.57 (2H, m), 10.17 (1H, s). LC-MS (ESI) calcd for C₂₅H₂₅FN₄O₆S₂ 560.12, found 561.3 [M+H⁺]. ee = 90% [HPLC-analysis: Chiralpak AS-RH 2.1 × 150 mm, 5 micron at r.t., Solvent A - Solvent B (see table for gradient), 0.3 mL/min, 312 nm, *t*1 = 4.3 min (major), *t*2 = 6.0 min].

Alternatively, *N*-{3-[(1*R,*2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide can be prepared as follows:

### f) (rac-di-exo)-3-Aza-tricyclo[4.2.1.0^{2,5}]nonan-4-one

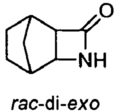

Bicyclo[2.2.1]hept-2-ene (1000 g, 10.6 mol) was dissolved in ethyl acetate (1.7 L) and the resulting solution was cooled to 0 °C. Chlorosulfonyl isocyanate (969 mL, 11.1 mol) was added at 0-20 °C over 30 min. The mixture was allowed to warm to 25 °C and stirred for 4 h, then cooled to 0 °C. A mixture of sodium sulfite (1500 g, 11.9 mol) in water (6 L) was added at 0-20 °C. The milky suspension was stirred at 25 °C for 30 min and cooled to 0 °C. A 50% aqueous sodium hydroxide solution (1.6 L, 30.3 mol) was added at 0-15 °C to adjust to pH 7. A saturated aqueous sodium carbonate solution (300 mL) was added to adjust the pH to 7.5-8.0. The mixture was filtered and the solid was washed with ethyl acetate (3 × 2 L) and the solid was discarded. The combined ethyl acetate extracts were washed with saturated aqueous brine solution (2 L), dried over magnesium sulfate and filtered. The solution was concentrated *in vacuo* to dryness to afford the desired product, (*rac*-di-*exo*)-3-aza-tricyclo[4.2.1.0^{2,5}]nonan-4-one (1220 g, 8.9 mol, 84%), as a white glassy solid. ¹H NMR (400 MHz, CDCl₃) δ 1.02 - 1.11 (2H, m), 1.24 (1H, dt, *J₁* = 10.9 Hz, *J₂* = 1.6 Hz), 1.51 - 1.72 (3H, m), 2.37 - 2.37 (1H, m), 2.43-2.44 (1H, m), 2.99-3.00 (1H, m), 3.40 (1H, d, *J* = 3.4 Hz), 5.73 (1H, bs).

### g) (rac-di-exo)-3-Amino-bicyclo[2.2.1]heptane-2-carboxylic acid hydrochloride

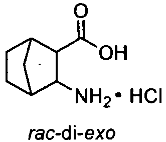

To (*rac*-di-*exo*)-3-aza-tricyclo[4.2.1.0^{2,5}]nonan-4-one (23.37 g, 170.4 mmol) was added a 12.0 M aqueous hydrochloric acid solution (150 mL). The mixture was stirred at 25 °C for 12 h. The solvent was evaporated *in vacuo* and the crude compound was dried under high vacuum for 0.5 h. The crude compound was triturated with acetone and filtered to afford (*rac*-di-*exo*)-3-amino-bicyclo[2.2.1]heptane-2-carboxylic acid hydrochloride (28.43 g, 148.3 mmol, 87%), as a white solid. ¹H NMR (400 MHz, DMSO-*d₆*) δ 1.15 - 1.26 (3H, m), 1.42 - 1.59 (2H, m), 1.87 (1H, d, *J* = 10.3 Hz), 2.33 (1H, d, *J=* 3.4 Hz), 2.45 (1H, d, *J=* 2.3 Hz), 2.67 (1H, d, J= 7.6 Hz), 3.23 - 3.26 (1H, m), 7.93 (3H, bs), 12.73 (1H, bs).

### h) (rac-di-exo)-3-Amino-bicyclo[2.2.1]heptane-2-carboxylic acid ethyl ester hydrochloride

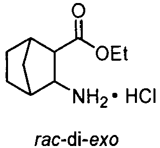

To absolute ethanol (75 mL) at -10 °C was added thionyl chloride (4.1 mL, 54.5 mmol) dropwise followed by (*rac*-di-*exo*)-3-amino-bicyclo[2.2.1]heptane-2-carboxylic acid hydrochloride (9.60 g, 50.1 mmol). The mixture was stirred at 0 °C for 1 h, at 25 °C for 4 h, and heated at reflux for 0.5 h. The solution was concentrated *in vacuo* and dried under high vacuum to afford the crude (*rac*-di-*exo*)-3-amino-bicyclo[2.2.1]heptane-2-carboxylic acid ethyl ester hydrochloride (11.01 g, 50.1 mmol, 100%), as an off-white solid. ¹H NMR (400 MHz, DMSO-*d₆*) δ 1.17 - 1.27 (3H, m), 1.21 (3H, t, *J=* 7.0 Hz), 1.43 - 1.57 (2H, m), 1.91 (1H, d, *J=* 10.0 Hz), 2.36 (1H, d, *J* = 3.9 Hz), 2.42 (1H, d, *J=* 3.0 Hz), 2.72 (1H, d, *J* = 7.6 Hz), 3.28 (1H, d, *J* = 8.3 Hz), 4.00 - 4.13 (2H, m), 8.06 (3H, bs).

### i) (rac-di-exo)-3-Amino-bicyclo[2.2.1]heptane-2-carboxylic acid ethyl ester

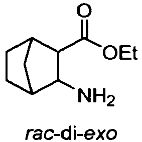

To (*rac-*di*-exo*)*-*3-amino-bicyclo[2.2.1]heptane-2-carboxylic acid ethyl ester hydrochloride (11.01 g, 50.1 mmol) was added saturated aqueous sodium bicarbonate solution (50 mL) and the mixture was stirred at 25 °C for 0.5 h. The crude product was extracted with ethyl acetate (3 × 100 mL). The solution was dried over magnesium sulfate, filtered, and concentrated *in vacuo* and dried under high vacuum for 2 h to afford the crude (*rac-*di*-exo*)-3-amino-bicyclo[2.2.1]heptane-2-carboxylic acid ethyl ester (8.17 g, 44.6 mmol, 89%), as a brown oil. ¹H NMR (400 MHz, CDCl₃) δ 1.10 1.26 (3H, m), 1.29 (3H, t, *J=* 7.0 Hz), 1.45 - 1.62 (2H, m), 1.86 (2H, bs), 1.95 (1H, dt, *J₁* = 10.3 Hz, *J₂* = 1.9 Hz), 2.09 (1H, d, *J=* 4.5 Hz), 2.49 (1H, d, *J=* 4.2 Hz), 2.56 (1H, d, *J=* 9.0 Hz), 3.24 (1H, d, *J* = 7.7 Hz), 4.09 - 4.21 (2H, m).

### j) (1R,2S,3R,4S)-3-Ethoxycarbonyl-bicyclo[2.2.1]hept-2-yl-aminium (1'S)-(+)-10-camphorsulfonate

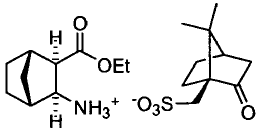

To a solution of (*rac*-di-*exo*)-3-amino-bicyclo[2.2.1]heptane-2-carboxylic acid ethyl ester (408.47 g, 2.98 mol) in ethyl acetate (500 mL) was added a solution of (1*S*)-(+)-10-camphorsulfonic acid (691.70 g, 2.98 mol) in ethanol (800 mL) at 50-75 °C over 30 min. The resulting solution was stirred at 70 °C for 1 h. More ethyl acetate (2.7 L) was added at >55 °C. The solution was allowed to cool to 50 °C and seeded with (1*R*,2*S*,3*R*,4*S*)-3-ethoxycarbonyl-bicyclo[2.2.1]hept-2-yl-aminium (1'*S*)-(+)-10-camphorsulfonate (ca. 20 mg). The mixture was allowed to cool to 25 °C and stirred for 16 h. The suspension was filtered and the wet filter cake was washed with ethyl acetate (2 × 500 mL). The crude salt was recrystallized from ethanol (600 mL) and ethyl acetate (3 L) to afford the desired product, (1*R*,2*S*,3*R*,4*S*)-3-ethoxycarbonyl-bicyclo[2.2.1]hept-2-yl-aminium (1'*S*)-(+)-10-camphorsulfonate (334.84 g, 0.806 mol, 27%, >99.5% de), as a white solid. ¹H NMR (400 MHz, CDCl₃) δ 0.84 (3H, s), 1.08 (3H, s), 1.30 (3H, t, *J=* 6.9 Hz), 1.32-1.43 (4H, m), 1.58-1.75 (3H, m), 1.89 (1H, d, *J=* 17.7 Hz), 1.95-2.07 (3H, m), 2.33 (1H, dt, *J₁* = 18.4 Hz, *J₂* = 3.9 Hz), 2.53 (1H, s), 2.58-2.65 (1H, m), 2.69 (1H, d, *J* = 2.9 Hz), 2.76-2.79 (2H, m), 3.26 (1H, d, *J* = 14.1 Hz), 3.60 (1H, d, *J =* 7.4 Hz), 4.14-4.27 (2H, m), 7.80 (3H, bs).

Alternatively, (1*R*,2*S*,3*R*,4*S*)-3-ethoxycarbonyl-bicyclo[2.2.1]hept-2-yl-aminium (1'*S*)-(+)-10-camphorsulfonate can be prepared as follows:
(*rac*-di-*exo*)-3-Aza-tricyclo[4.2.1.0^{2,5}]nonan-4-one (prepared as described in Example 2f, 1220 g, 8.9 mol) was dissolved in ethyl acetate (1.7 L). The solution was heated to 50 °C and a solution of (1*S*)-(+)-10-camphorsulfonic acid (2066 g, 8.9 mol) in ethanol (2.5 L) at 50-75 °C over 30 min. The resulting solution was stirred at 70 °C for 2 h. More ethyl acetate (8 L) was added causing the temperature to drop to >55 °C and the solution was seeded with (1*R*,2*S*,3*R*,4*S*)-3-ethoxycarbonyl-bicyclo[2.2.1]hept-2-yl-aminium (1'*S*)-(+)-10-camphorsulfonate (ca. 100 mg). The mixture was allowed to cool to 25 °C and stirred for 16 h. The precipitate was collected by filtration and the wet filter cake was washed with ethyl acetate (2 × 2 L). The crude salt was dried at 25 °C for 48 h and then was recrystallized from ethanol (2 L) and ethyl acetate (2.5 L) to afford the desired product, (1*R*,2*S*,3*R*,4*S*)-3-ethoxycarbonyl-bicyclo[2.2.1]hept-2-yl-aminium (1'*S*)-(+)-10-camphorsulfonate (920 g, 2.21 mol, 25%, >99.9% de), as a white solid.

### k) (1S,2R,3S,4R)-3-Amino-bicyclo[2.2.1]heptane-2-carboxylic acid ethyl ester

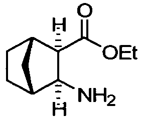

To (1*R*,2*S*,3*R*,4*S*)-3-ethoxycarbonyl-bicyclo[2.2.1]hept-2-yl-aminium (1'*S*)-(+)-10-camphorsulfonate (2.76 g, 6.64 mmol) was added ethyl acetate (28 mL) and saturated aqueous sodium carbonate solution (28 mL) and the mixture was stirred at 25 °C for 0.5 h. The organic layer was separated and the aqueous layer was extracted with ethyl acetate (2 × 50 mL). The solution was dried over magnesium sulfate, filtered, and concentrated *in vacuo* and dried under high vacuum for 1 h to afford (1*S*,2*R*,3*S*,4*R*)-3-amino-bicyclo[2.2.1]heptane-2-carboxylic acid ethyl ester (1.15 g, 6.28 mmol, 95%), as a colorless oil. ¹H NMR (400 MHz, CDCl₃) δ 1.10-1.26 (3H, m), 1.29 (3H, t, *J* = 7.0 Hz), 1.45 - 1.62 (2H, m), 1.86 (2H, bs), 1.95 (1H, dt, *J₁* = 10.3 Hz, *J*₂ = 1.9 Hz), 2.09 (1 H, d, *J* = 4.5 Hz), 2.49 (1H, d, *J =* 4.2 Hz), 2.56 (1H, d, *J=* 9.0 Hz), 3.24 (1H, d, *J=* 7.7 Hz), 4.09 - 4.21 (2H, m).

In order to determine the enantiomeric excess, (1*S*,2*R*,3*S*,4*R*)-3-amino-bicyclo[2.2.1]heptane-2-carboxylic acid ethyl ester was derivatized to the (S)-mandelate salt as follows: To a solution of (1*S*,2*R*,3*S*,4*R*)-3-amino-bicyclo[2.2.1]heptane-2-carboxylic acid ethyl ester (34.2 mg, 0.187 mmol) in ethyl acetate (1 mL) was added (S)-α-hydroxyphenylacetic acid (28.7 mg, 0.187 mmol) and the mixture was stirred at 25 °C for 0.5 h. The solid was filtered and dried under high vacuum to afford (1*R*,2*S*,3*R*,4*S*)-3-ethoxycarbonyl-bicyclo[2.2.1]hept-2-yl-aminium (*S*)-α-hydroxyphenylacetate (11.4 mg, 0.034 mmol, 18%, de = 97%), as a white solid. ¹H NMR (400 MHz, CDCl₃) δ 1.08 - 1.20 (3H, m), 1.28 (3H, t, *J=* 7.1 Hz), 1.50-1.59 (2H, m), 1.79 (1H, d, *J* = 10.9 Hz), 2.23 (1H, s), 2.46 - 2.48 (2H, m), 3.04 (1H, d, *J=* 7.8 Hz), 4.05 - 4.18 (2H, m), 4.89 (1H, s), 5.49 (3H, bs), 7.22 - 7.31 (3H, m), 7.43 (2H, d, *J* = 6.9 Hz).

### 1) (1S,2R,3S,4R)-3-(4-Fluorobenzylamino)-bicyclo[2.2.1]heptane-2-carboxylic acid ethyl ester

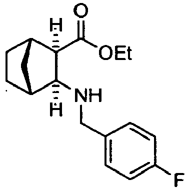

To a solution of (1*S*,2*R*,3*S*,4*R*)-3-amino-bicyclo[2.2.1]heptane-2-carboxylic acid ethyl ester (1.15 g, 6.28 mmol) in ethanol (30 mL) was added 4-fluorobenzaldehyde (0.68 mL, 6.31 mmol), glacial acetic acid (0.4 mL, 6.99 mmol), and sodium cyanoborohydride (1.04 g, 15.7 mmol) at 25 °C. After stirring for 3 h, the mixture was diluted with ethyl acetate (50 mL) and quenched with saturated aqueous sodium bicarbonate solution (50 mL) for 0.5 h. The mixture was filtered through Celite. The organic layer was separated and the aqueous layer was extracted with ethyl acetate (2 × 50 mL). When all solvent was removed, a solid was formed. The solid was filtered, washed with water, and dried *in vacuo* to afford the desired product, (1*S*,2*R*,3*S*,4*R*)-3-(4-fluorobenzylamino)-bicyclo[2.2.1]heptane-2-carboxylic acid ethyl ester (1.74 g, 5.97 mmol, 95%), as a white solid. ¹H NMR (400 MHz, CDCl₃) δ 1.05 - 1.16 (2H, m), 1.21 (1H, dt, *J₁* = 8.0 Hz, *J₂* = 1.6 Hz), 1.27 (3H, t, *J* = 7.4 Hz), 1.45 - 1.61 (2H, m), 1.94 (1H, dt, *J₁ =* 10.1 Hz, *J₂* = 1.9 Hz), 2.28 (1H, d, *J* = 3.9 Hz), 2.43 (1H, d, *J* = 3.3 Hz), 2.60 (1H, dd, *J₁* = 8.8 Hz, *J₂* = 1.5 Hz), 2.94 (1H, d, *J* = 7.8 Hz), 3.66 (1H, d, *J* = 13.2 Hz), 3.80 (1H, d, *J=* 13.5 Hz), 4.13 (2H, q, *J=* 7.0 Hz), 6.97 (2H, t, *J=* 8.5 Hz), 7.26 (2H, t, *J* = 7.1 Hz).

Alternatively, (1*S*,2*R*,3*S*,4*R*)-3-(4-Fluorobenzylamino)-bicyclo[2.2.1]heptane-2-carboxylic acid ethyl ester can be prepared as follows:

(1*R*,2*S*,3*R*,4*S*)-3-ethoxycarbonyl-bicyclo[2.2.1]hept-2-yl-aminium (1'*S*)-(+)-10-camphorsulfonate (prepared as described in Example 2j, 2000 g, 4.81 mol) and powdered potassium carbonate (1320 g, 9.62 mol) were suspended in ethyl acetate (20 L). The suspension was stirred at 25 °C for 16 h and filtered. The ethyl acetate filtrate was concentrated *in vacuo* to afford the free amine (1050 g) as a liquid. The liquid was dissolved in ethanol (10 L), and 4-fluorobenzaldehyde (558 mL, 5.3 mol) and acetic acid (362 mL, 6.3 mol) were added, causing the temperature to rise to 28-30 °C. The solution was allowed to cool to 25 °C and stirred for 30 min. A cloudy solution of sodium cyanoborohydride (756 g, 12.03 mol) in ethanol (5 L) was added in 20 min, causing the temperature to rise to 45-50 °C. The mixture was allowed to cool to 25 °C and stirred for 16 h. The mixture was concentrated *in vacuo* to a volume of about 13-14 L. Water (1-2 L) was added, and the resulting mixture was further concentrated *in vacuo.* A saturated aqueous sodium bicarbonate solution (4 L) and water (4 L) were added with stirring. The pH was adjusted to 8.0-8.5 by adding additional saturated aqueous sodium bicarbonate solution (∼500 mL). The mixture was stirred for 1 h before the solids were collected by filtration and the wet filter cake was washed with water (2 L). The solid was dried *in vacuo* at 35 °C for 64 h to afford the desired product, (1*S*,2*R*,3*S*,4*R*)-3-(4-fluoro-benzylamino)-bicyclo[2.2.1]heptane-2-carboxylic acid ethyl ester (1350 g, 4.63 mol, 96%), as a white solid.

### m) (1S,2R,3S,4R)-3-{(4-Fluorobenzyl)-[2-(7-methanesulfonylamino-1,1-dioxo-1,4-dihydro-1λ⁶-benzo[1,2,4]thiadiazin-3-yl)-acetyl]-amino}-bicyclo[2.2.1]heptane-2-carboxylic acid ethyl ester

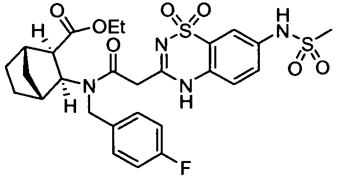

To a solution of (1*S,*2*R*,3*S*,4*R*)-3-(4-fluorobenzylamino)-bicyclo[2.2.1]heptane-2-carboxylic acid ethyl ester (100.6 mg, 0.345 mmol) in *N,N-*dimethylformamide (3.0 mL) was added (7-methanesulfonylamino-1,1-dioxo-1,4-dihydro-1λ⁶-benzo[1,2,4]thiadiazin-3-yl)-acetic acid (prepared as described in Example 1, 120.8 mg, 0.362 mmol), 4-dimethylaminopyridine (10.6 mg, 0.086 mmol), and 1-[3-(dimethylamino)propyl]-3-ethylcarbodiimide hydrochloride (70.9 mg, 0.362 mmol). After stirring at 25 °C for 12 h, the mixture was diluted with ethyl acetate and acidified with 1.0 M aqueous hydrochloric acid solution to pH 1. The organic layer was separated and the aqueous layer was extracted with ethyl acetate (2 × 20 mL). The combined organic layer was dried over magnesium sulfate, filtered, and concentrated *in vacuo,* and dried under high vacuum to afford the crude product, (1*S*,2*R*,3*S*,4*R*)-3-{(4-fluorobenzyl)-[2-(7-methanesulfonylamino-1,1-dioxo-1,4-dihydro-1λ⁶-benzo[1,2,4]thiadiazin-3-yl)-acetyl]-amino}-bicyclo[2.2.1]heptane-2-carboxylic acid ethyl ester, as a faintly yellow oil. The crude product was used in the next step without further purification. LC-MS (ESI) calcd for C₂₇H₃₁FN4O₇S₂ 606.16, found 607.2 [M+H⁺].

### n) N-{3-[(1R,2S,7R,8S)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide

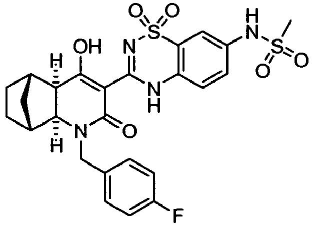

To a solution of the crude (1*S*,2*R*,3*S*,4*R*)-3-{(4-fluorobenzyl)-[2-(7-methanesulfonylamino-1,1-dioxo-1,4-dihydro-1λ⁶-benzo[1,2,4]thiadiazin-3-yl)-acetyl]-amino}-bicyclo[2.2.1]heptane-2-carboxylic acid ethyl ester in absolute ethanol (3 mL) was added a 21 wt. % solution of sodium ethoxide in ethanol (0.51 mL, 1.37 mmol). After stirring at 60 °C for 2 h, the mixture was diluted with ethyl acetate and acidified with 1.0 M aqueous hydrochloric acid solution to pH 1. The organic layer was separated and the aqueous layer was extracted with ethyl acetate (2 × 20 mL). The combined organic layer was dried over magnesium sulfate, filtered, and concentrated *in vacuo.* The crude mixture was purified by flash column chromatography (Teledyne Isco RediSep column; 0 to 100% ethyl acetate in hexanes) to afford the desired product, *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (131.5 mg, 0.235 mmol, 68% over two steps), as an off-white solid. ¹H NMR (400 MHz, CD₃OD) δ 1.28 (2H, d, *J=* 11.0 Hz), 1.47 (1H, t, *J* = 10.8 Hz), 1.57 - 1.74 (3H, m), 2.56 (1H, d, *J= 3.2* Hz), 2.75 (1H, d, *J* = 2.3 Hz), 2.96 (1H, d, *J* = 9.2 Hz), 3.02 (3H, s), 3.58 (1H, d, *J=* 9.2 Hz), 4.42 (1H, d, *J=* 15.5 Hz), 5.03 (1H, d, *J* = 15.7 Hz), 7.04 (2H, t, *J* = 8.5 Hz), 7.31 (2H, dd, *J₁* = 7.9 Hz, *J₂* = 5.5 Hz), 7.37 (1H, d, *J* = 8.8 Hz), 7.54 (1H, dd, *J₁* = 8.3 Hz, *J₂* = 2.3 Hz), 7.69 (1H, d, *J=* 2.3 Hz). LC-MS (ESI) calcd for C₂₅H₂₅FN₄O₆S₂ 560.12, found 561.4 [M+H⁺]. ee = 98.5% [HPLC-analysis: Chiralpak AS-RH 2.1 × 150 mm, 5 micron at r.t., Solvent A - Solvent B (see table for gradient), 0.3 mL/min, 312 nm, *t*1 = 7.58 min (major), *t*2 = 8.95 min].

Alternatively, *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide can be prepared as follows:

(7-Methanesulfonylamino-1,1-dioxo-1,4-dihydro-1λ⁶-benzo[1,2,4]thiadiazin-3-yl)-acetic acid (prepared as described in Example 1g, 1.88 kg, 5.63 mol) and (1*S*,2*R*,3*S*,4*R*)-3-(4-fluoro-benzylamino)-bicyclo[2.2.1]heptane-2-carboxylic acid ethyl ester (prepared as described in Example 21, 1.72 kg, 5.91 mol) were dissolved in acetonitrile (18.8 L) at 23 °C. *N*-Methylmorpholine (1.25 kg, 12.4 mol) was added and the resulting suspension was stirred at 23 °C for 1 h. The suspension was cooled to 0 °C and 1-[3-(dimethylamino)propyl]-3-ethylcarbodiimide hydrochloride (1.19 kg, 6.20 mol) was added in one portion. The mixture was stirred at 0 °C for 3 h, and was then allowed to warm to 23 °C and stirred overnight. Triethylamine (1.88 kg, 18.6 mol) was added and the mixture was then heated at 50 °C for 3 h. The mixture was partially concentrated *in vacuo* at 45 °C, and was then diluted with ethyl acetate (22.5 L) and washed with 2.0 M aqueous hydrochloric acid solution (22.6 L). The resulting aqueous fraction was extracted with ethyl acetate (2 × 9.4 L). The combined organic extracts were washed with 1.0 M aqueous hydrochloric acid solution (10.4 L) and then with water (18.8 L). The resulting organic fraction was filtered through Celite (600 g), and the filtrate was then partially concentrated *in vacuo* at 45 °C. Absolute ethanol (5.6 L) was added to the residue, and the mixture was then heated at 50 °C with stirring. Dichloromethane (400 mL) was added in portions until crystallization initiated. Absolute ethanol (20.7 L) was added in portions over 1 h, and the resulting mixture was stirred at 23 °C overnight. The mixture was filtered and the solid was then washed with absolute ethanol (1.9 L). The solid was further dried *in vacuo* at 45 °C to afford the desired product, *N-*{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (2.46 kg, 4.39 mol, 78%), as an off-white crystalline solid.

### Example 3: N-{3-[(1R,2S,7R,8S)-3-(4-Fluoro-benzyl)-6-ydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide,L-arginine salt

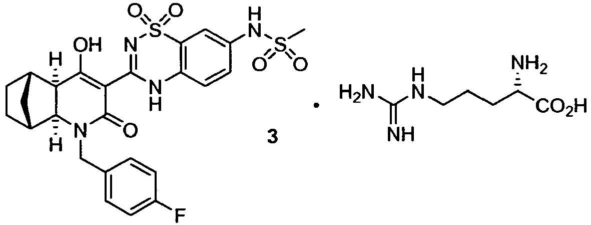

*N*-{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (prepared as described in Example 2, 0.280 g, 0.499 mmol) was dissolved in acetonitrile (5.0 mL). A 0.1 M aqueous L-arginine solution (3.0 mL, 0.3 mmol) was added, which was followed by addition of a 0.1 M solution ofL-arginine in 1-propanol (2.0 mL, 0.2 mmol). After stirring for 6 h at 23 °C, the flask was opened to the atmosphere and the suspension was stirred for 16 h. The solid was collected by filtration and further dried *in vacuo* at 23 °C to afford the desired product, *N*-{3-[(1*R*,2*S*,7*R*,8*S*)-3-(4-fluoro-benzyl)-6-hydroxy-4-oxo-3-aza-tricyclo[6.2.1.0.^{2,7}]undec-5-en-5-yl]-1,1-dioxo-1,4-dihydro-1λ⁶-benzo[1,2,4]thiadiazin-7-yl}-methanesulfonamide, *L*-arginine salt, monohydrate (0.257 g, 0.341 mmol, 68%), as a crystalline solid. ¹H NMR (300 MHz, DMSO-d₆) δ: 0.96 - 1.17 (2H, m), 1.28 (1H, app t, *J=* 10.0 Hz), 1.35 - 1.82 (7H, m), 2.33 (1H, app d, J= 3.0 Hz), 2.43 (1H, d, *J* = 9.3 Hz), 2.97 (3H, s), 3.00 - 3.17 (2H, m), 3.23 (1H, d, *J* = 9.3 Hz), 4.21 (1H, d, *J* = 15.3 Hz), 4.94 (1H, d, *J =* 15.3 Hz), 7.04 - 7.15 (3H, m), 7.27 (2H, dd, *J* = 5.7, 8.7 Hz), 7.35 (1H, dd, *J=* 2.5, 8.9 Hz), 7.35 - 7.51 (4H, m), 8.82 (1H, br s), 15.29 (1H, br s). Anal. calcd for C₃₁H₃₉FN₈O₈S₂ • H₂O: C, 49.46; H, 5.49; N, 14.88; O, 19.13; S, 8.52; F, 2.52; found: C, 49.49; H, 5.23; N, 14.96; O, 18.69; S, 8.82; F, 2.81. m.p. = 216 °C (DSC).

### Example 4: N-{3-[(1R,2S,7R,8S)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, L-lysine salt

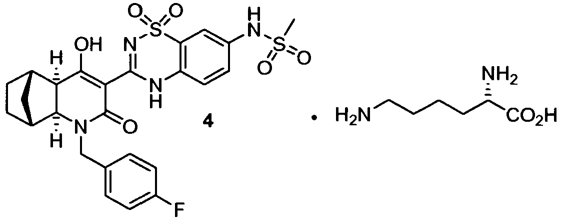

*N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (prepared as described in Example 2, 0.090 g, 0.160 mmol) was dissolved in acetonitrile (2.5 mL). An aqueous solution of *L*-lysine (0.469 mL of a 50 mg/mL solution in water, 0.160 mmol) was added. The solvent was allowed to evaporate under a flow of nitrogen and ethanol (0.5 mL) was added. The mixture was stirred at 35 °C for 2 d, and was then immersed in an ultrasonic bath. Water (0.5 mL) was added, and the mixture was stirred at 23 °C for 3 d. The solid was collected by filtration and further dried *in vacuo* at 23 °C to afford the desired product, N-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, *L*-lysine salt, monohydrate (0.070 g, 0.096 mmol, 60%), as a crystalline solid. ¹H NMR (300 MHz, DMSO-d₆) δ: 0.96 - 1.15 (2H, m), 1.22 - 1.76 (10H, m), 2.34 (1H, app d, *J=* 2.7 Hz), 2.43 (1H, d, *J* = 9.3 Hz), 2.74 - 2.78 (2H, m), 2.97 (3H, s), 3.18 - 3.29 (1H, m), 4.21 (1H, d, *J=* 15.3 Hz), 4.95 (1H, d, *J* = 15.6 Hz), 7.07 - 7.18 (3H, m), 7.27 (2H, dd, *J=* 5.7, 8.7 Hz), 7.36 (1H, dd, *J* = 2.4, 8.7 Hz), 7.44 (1H, d, *J =* 2.4 Hz), 15.31 (1H, br s). Anal. calcd for C₃₁H₃₉FN₆O₈S₂ • H₂O: C, 51.37; H, 5.70; N, 11.59; O, 19.87; S, 8.85; F, 2.62; found: C, 51.13; H, 5.52; N, 11.63; O, 20.07; S, 9.20; F, 2.71. m.p. = 200 °C (DSC).

### Example 5: N-{3-[(1R,2S,7R,8S)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, hemi magnesium salt

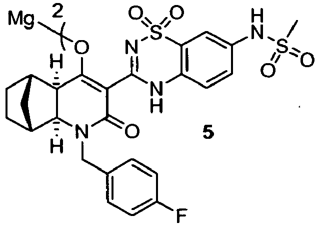

*N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (prepared as described in Example 2, 0.465 g, 0.829 mmol) was dissolved in acetone (9.0 mL). A 7-8 wt. % solution of magnesium methoxide in methanol (0.593 mL, 0.414 mmol) was added. The solvent was evaporated, and the residue was then diluted with water (0.9 mL) and acetone (1.8 mL). The resulting mixture was stirred at 23 °C for 16 h. The solid was collected by filtration and further dried *in vacuo* at 23 °C to afford the desired product, *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, hemi magnesium salt, trihydrate (0.377 g, 0.602 mmol, 73%), as a crystalline solid. ¹H NMR (300 MHz, DMSO-d₆) δ: 0.96 - 1.17 (2H, m), 1.22 - 1.58 (4H, m), 2.33 (1H, br s), 2.44 (1H, d, *J* = 9.6 Hz), 2.98 (3H, s), 3.23 (1H, d, *J=* 9.3 Hz), 4.21 (1H, d, *J* = 14.7 Hz), 4.94 (1H, d, *J=* 15.3 Hz), 7.03 - 7.19 (3H, m), 7.21 - 7.48 (4H, m), 9.81 (1H, brs), 15.35 (1H, brs). Anal. calcd for C₂₅H₂₄N₄O₆FS₂ • 0.5 Mg • 3 H₂O: C, 47.98; H, 4.83; N, 8.95; O, 23.01; S, 10.25; F, 3.04; Mg, 1.94; found: C, 47.66; H, 4.89; N, 8.98; O, 23.00; S, 11.36; F, 3.09; Mg, 1.82. m.p. = 184 °C (DSC).

### Example 6: N-{3-[(1R,2S,7R,8S)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, sodium salt

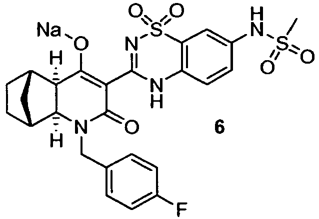

*N-*{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (prepared as described in Example 2, 0.407 g, 0.726 mmol) was suspended in ethanol (11.0 mL). A 1.0 M aqueous sodium hydroxide solution (0.726 mL, 0.726 mmol) and water (1.0 mL) were added. The mixture was seeded with a crystal of N-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, sodium salt (produced from a separate batch), and the mixture was then stirred at 23 °C for 1 d. The solid was collected by filtration and further dried *in vacuo* at 23 °C to afford the desired product, *N*-{3-[(1*R*,2*S*,7*R*,8*S*)-3-(4-fluoro-benzyl)-6-hydroxy-4-oxo-3-aza-tricyclo[6.2.1.0²,⁷]undec-5-en-5-yl]-1,1-dioxo-1,4-dihydro-1λ⁶-benzo[1,2,4]thiadiazin-7-yl}-methanesulfonamide, sodium salt, hydrate (2.25 molar equiv. water) (0.235 g, 0.377 mmol, 52%), as a crystalline solid. ¹H NMR (300 MHz, DMSO-d₆) δ: 0.99 - 1.11 (2H, m), 1.28 (1H, app t, *J* = 10.2 Hz), 1.36 - 1.53 (3H, m), 2.33 (1H, app d, *J=* 2.7 Hz), 2.42 (1H, d, *J=* 9.3 Hz), 2.97 (3H, s), 3.22 (1H, d, *J=* 9.3 Hz), 4.20 (1H, d, *J* = 15.3 Hz), 4.95 (1H, d, *J* = 15.3 Hz), 7.09 - 7.16 (3 H, m), 7.25 - 7.36 (3H, m), 7.42 (1H, d, *J* = 2.4 Hz), 9.79 (1H, s), 15.32 (1H, s). Anal. calcd for C₂₅H₂₄FN₄NaO₆S₂ • 2.25 H₂O: C, 48.19; H, 4.61; N, 8.99; O, 21.18; S, 10.29; F, 3.05; Na, 3.69; found: C, 48.14; H, 4.67; N, 8.97; O, 21.07; S, 10.25; F, 3.13; Na, 3.87. m.p. = 182-188 °C (DSC).

### Example 7: N-{3-[(1R,2S,7R,8S)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, potassium salt

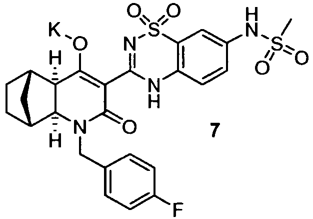

*N*-{3-[(1*R*,2*S*,7*R*,8*S*)-3-(4-Fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide (prepared as described in Example 2, 0.281 g, 0.501 mmol) was dissolved in methyl ethyl ketone (8.0 mL). A 0.5 M aqueous potassium hydroxide solution (1.0 mL, 0.500 mmol) was added. The solution was seeded with crystalline *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, potassium salt (produced from a separate batch), and the resulting mixture was then stirred at 23 °C for 3 h. The solid was collected by filtration and further dried *in vacuo* at 23 °C to afford the desired product, *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, potassium salt, hydrate (0.75 molar equiv. water) (0.127 g, 0.207 mmol, 41%), as a crystalline solid. ¹H NMR (300 MHz, DMSO-d₆) δ: 0.99 - 1.11 (2H, m), 1.27 (1H, app t, *J* = 10.3 Hz), 1.36 - 1.54 (3H, m), 2.33 (1H, br s), 2.42 (1H, d, *J* = 9.0 Hz), 2.95 (3H, s), 3.22 (1H, d, *J=* 9.3 Hz), 4.20 (1H, d, *J=* 15.3 Hz), 4.96 (1H, d, *J=* 15.6 Hz), 7.09 - 7.15 (3H, m), 7.25-7.34 (3H, m), 7.41 (1H, d, *J=* 2.7 Hz), 9.84 (1H, br s), 15.30 (1H, s). Anal. calcd for C₂₅H₂₄FKN₄O₆S₂ • 0.75 H₂O: C, 49.05; H, 4.20; N, 9.15; O, 17.64; S, 10.48; F, 3.10; K, 6.39; found: C, 48.82; H, 4.11; N, 9.06; O, 17.35; S, 10.37; F, 3.18; K, 6.75. m.p. = 278 °C (DSC).

### BIOLOGICAL TESTING

The ability of compounds to inhibit HCV replication can be demonstrated in the following *in vitro* assays.

### LUCIFERASE-BASED HCV REPLICON ASSAY PROTOCOL

The cell culture component of the assay is performed essentially as described by Bartenschlager et al., Hepatology, 2002, 35, 694-703, wherein exponentially growing HCV Huh-luc/neo-ET cells are seeded at 6 × 10³ cells/well in 96 well assay plate. 24 hours later the cells are treated with various concentrations of compound or combination of compounds in triplicate using both fixed ratios and checkerboard matrices of test agents and cultured for 72 hours. The luciferase activity in the wells is determined using Bright-Glo reagent (Promega, Madison, Wisconsin) with a luminometer (Wallac 1420 Multilabel HTS Counter Victor 2). The background control is replicon cells treated with 100 nM BILN-2061, an inhibitor of the HCV protease. % Inhibition is determined for each compound concentration or combination of compounds in relation to the negative (no compound) control to calculate the EC₅₀. In the case of ANA773, conditioned media from human PBMCs from multiple donors is pooled after treatment with 100 µM of the active of ANA773 for 24 hours.

### EVALUATION OF COMBINATIONS OF AGENTS

Compound **2** was evaluated in combination with Interferon α-2a, Telaprevir (HCV NS3/4 protease inhibitor, also known as VX-950), PSI-6130 the active agent of R7128 (HCV NS5B nucleoside inhibitor), and ANA773 (TLR7 agonist). The 50% inhibitory concentration (EC₅₀) of each compound or combination of compounds is determined independently to determine the range of concentrations used to characterize the interaction of two agents in inhibiting the HCV replicon. Each compound is tested singly and in combination at two- or three-fold serial dilutions above and below the EC₅₀. The ratio of the two compounds tested either remained fixed or is varied across the dosing range to explore the greatest combination surface.

The combination data is analyzed using CalcuSyn (Biosoft, Ferguson, Mo.), a computer program based on the method of Chou and Talalay, J. Biol. Chem., 1977, 252, 6438-6442. Combination index (CI) values for each experimental combination are calculated at the EC₅₀, EC₇₅ and EC₉₀ levels. CI values of <1, 1, and >1 indicate synergy, an additive effect, and antagonism, respectively.

In general, combinations that are broadly antagonistic are not suitable for combination therapy in vivo. Combinations that are synergistic or additive may offer greater benefit then expected by the action of the individual agents alone. Combination of Compound 2 with Interferon α-2a (IFN), a component of the current standard of care, results in a substantial increase in potency compared to each agent dosed independently (Figure 1). Analysis of the combination data by CalcuSyn demonstrates a synergistic interaction with a CI index of 0.2 ±0.04 (Table 1).

**Table 1. Combination effects of Compound 2 with other agents**

| Agents^{a} | CalcuSyn Combination Index (CI)^{b} |
|---|---|
| | EC₉₀ ± SD |
| IFN/Compound **2** | 0.2 ±0.04 |
| PSI-6130/Compound **2** | 0.5 ±0.2 |
| Telaprevir/Compound **2** | 0.7 ±0.4 |
| ANA773/Compound **2** | 0.8 ±0.1 |

| | |
|---|---|
| ^{a}Fixed concentration ratios reported are conditions where the initial concentrations of both agents are 10-fold above their EC₅₀ values. ^{b}CI values between 0.90 and 1.10 = additive; <0.90 = synergy; > 1.10 = antagonism | |

Combination of Compound **2** with the direct antiviral agents, PSI-6130 and Telaprevir are shown in Figures 2 and 3 where the dose response of PSI-6130 and Telaprevir are each evaluated in the presence of fixed concentrations of Compound **2.** The dose response curves in Figures 2a and 3a illustrate that at low concentrations of PSI-6130 or Telaprevir, the %Inhibition is dictated by the amount of Compound **2** present. Normalization of the inhibition data allows the effect of Compound **2** on the activity of the other direct antiviral agents to be clearly observed. At concentrations readily achieved clinically, the presence of Compound 2 increases the potency of both PSI-6130 (4-5 fold) and Telaprevir (2-3 fold), see Figures 2b, 3b, and Table 2.

**Table 2. Antiviral Effect of PSI-6130 and Telaprevir Combined with Compound 2**

| Compound **2** (ng/mL) | EC₉₀, µM |
|---|---|
| | PSI-6130 |
| 0 | 11.6 |
| 6 | 6.0 |
| 17 | 2.4 |

| | Telaprevir |
|---|---|
| 0 | 2.8 |
| 6 | 2.4 |
| 17 | 1.9 |
| 170 | 1.3 |

Evaluation of the combination data of Compound **2** with PSI-6130, Telaprevir and ANA773 by CalcuSyn demonstrate synergistic interactions with CI indices of <1 determined for all combinations (see Table 1).

## Claims

1. An *in vitro* method of inhibiting hepatitis C virus replication comprising exposing hepatitis C virus to a therapeutically effective amount of a composition comprising *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, or a salt or hydrate thereof, and a composition comprising one or more additional antiviral compounds selected from the group consisting of: VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, and ANA773.

2. The method of claim 1, wherein the hepatitis C virus is in a human cell.

3. The method of claims 1 or 2, wherein said one or more additional antiviral compounds is PSI-6130.

4. A combination of (i) a composition comprising *N*-{3-[(1*R*,2*S*,7*R*,8*S*)-3-(4-fluoro-benzyl)-6-hydroxy-4-oxo-3-aza-tricyclo[6.2.1.^{2,7}]undec-5-en-5-yl]-1,1-dioxo-1,4-dihydro-1λ⁶-benzo[1,2,4]thiadiazin-7-yl}-methanesulfonamide, a salt or hydrate thereof, and (ii) a composition comprising one or more additional antiviral compounds selected from the group consisting of: VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, and ANA773 for use in treating or preventing hepatitis C virus infection.

5. The combination for use according to claim 4, wherein the subject is a human.

6. The combination for use according to claims 4 or 5, wherein in the use in treating or preventing hepatitis C virus infection the compositions are administered separately.

7. The combination for use of claims 4-6, wherein said one or more additional antiviral compounds are selected from MK-7009, TMC-435350, BI-201335, PF-00868554, IDX-184, RG7128, PSI-6130, BMS-790052, and ANA773.

8. The combination for use of claim 7, wherein said one or more additional antiviral compounds is PSI-6130.

9. A pharmaceutically acceptable composition comprising a therapeutically effective amount of a composition comprising *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]-thiadiazin-7-yl}-methanesulfonamide, or a salt or hydrate thereof, and one or more additional antiviral compounds selected from the group consisting of: VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, and ANA773 and a pharmaceutically acceptable carrier.

10. The composition of claim 9, wherein said one or more additional antiviral compounds are selected from MK-7009, TMC-435350, BI-201335, PF-00868554, IDX-184, RG7128, PSI-6130, BMS-790052, and ANA773.

11. The composition of claim 10, wherein said one or more additional antiviral compounds is PSI-6130.

12. The composition of claims 9 to 11, wherein the salt of *N*-{3-[(1*R*,2*S*,7*R*,8*S*)-3-(4-fluorobenzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide is selected from the group consisting of: *L*-arginine, *L*-lysine, hemi-magnesium, sodium and potassium.

13. The combination for use of claim 4, wherein the combination is a combination of (i) a composition comprising *N-*{3-[(1*R,*2*S,*7*R,*8*S*)*-*3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamide, and (ii) a composition comprising PSI-6130.

14. The combination for use according to claim 13, wherein the subject is a human.

15. The combination for use according to claims 13 or 14, wherein in the use in treating or preventing hepatitis C virus infection the compositions are administered separately.

## Patentansprüche

1. *Ein in vitro* Verfahren zur Hemmung der Hepatitis-C-Virus-Replikation, umfassend das Hepatitis-C-Virus einer therapeutisch wirksamen Menge einer Zusammensetzung auszusetzen, die *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methansulfonamid oder ein Salz oder Hydrat davon umfasst, sowie einer Zusammensetzung, die eine oder mehrere zusätzliche antivirale Verbindungen umfasst, die aus der Gruppe ausgewählt werden, die aus: VBY-376, BMS-650032, MK-7009, TMC-435350, BI-201335, GS-9190, MK- 3281, VCH-759, VCH-916, ABT-333, BMS-791325, PF-00868554, IDX-184, RG7128, PSI-6130, BMS-790052 und ANA773 besteht.

2. Verfahren nach Anspruch 1, wobei das Hepatitis C-Virus in einer menschlichen Zelle vorkommt.

3. Verfahren nach den Ansprüchen 1 oder 2, wobei die eine oder mehreren zusätzlichen antiviralen Verbindungen PSI-613 ist.

4. Eine Kombination aus (i) einer Zusammensetzung, die *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methansulfonamid oder ein Salz oder Hydrat davon umfasst, und (ii) eine Zusammensetzung, die eine oder mehrere zusätzliche antivirale Verbindungen umfasst, die aus der Gruppe ausgewählt werden, die aus: VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, und ANA773 besteht, zur Behandlung oder Vorbeugung einer Hepatitis-C-Virus-Infektion.

5. Kombination zur Verwendung nach Anspruch 5, wobei das Subjekt ein Mensch ist.

6. Kombination zur Verwendung nach den Ansprüchen 4 oder 5, wobei bei der Verwendung bei der Behandlung oder Verbeugung der Hepatitis-C-Virus-Infektion die Zusammensetzungen getrennt verabreicht werden.

7. Die Kombination zur Verwendung nach Ansprüchen 4-6, wobei die eine oder mehrere zusätzliche antivirale Verbindungen von MK-7009, TMC-435350, BI-201335, PF-00868554, IDX-184, RG7128, PSI-6130, BMS-790052 und ANA773 gewählt werden.

8. Die Kombination zur Verwendung nach Anspruch 7, wobei die eine oder mehreren zusätzlichen antivirale Verbindungen PSI-6130 ist.

9. Pharmazeutisch akzeptable Zusammensetzung, die eine therapeutisch wirksame Menge einer Zusammensetzung umfasst, die *N*-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methansulfonamid oder ein Salz oder Hydrat davon umfasst, und eine oder mehrere zusätzliche antivirale Verbindungen, die aus der Gruppe ausgewählt werden, die aus: VBY-376, BMS-650032, MK-7009, TMC-435350, BI-201335, GS-9190, MK-3281, VCH -759, VCH-916, ABT-333, BMS-791325, PF-00868554, IDX-184, RG7128, PSI-6130, BMS-790052 und ANA773 besteht, und einen pharmazeutisch akzeptablen Träger.

10. Zusammensetzung nach Anspruch 10, wobei die ein oder mehrere zusätzlichen antiviralen Verbindungen aus MK-7009, TMC-435350, BI-201335, PF-00868554, IDX-184, RG7128, PSI-6130, BMS-790052 und ANA773 ausgewählt werden.

11. Zusammensetzung nach Anspruch 10, wobei die ein oder mehreren zusätzlichen antiviralen Verbindungen PSI-6130 ist.

12. Zusammensetzung nach den Ansprüchen 9 bis 11, wobei das Salz von *N-*{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methanesulfonamid aus der Gruppe ausgewählt wird, die aus L-Arginin, L-Lysin, hemi-Magnesium, Natrium und Kalium besteht.

13. Die Kombination zur Verwendung nach Anspruch 4, wobei die Kombination eine Kombination ist aus (i) einer Zusammensetzung, die N-{3-[(1*R*,2*S*,7*R*,8*S*)-3-(4-fluoro-benzyl)-6-hydroxy-4-oxo-3-aza-tricyclo[6.2.1.0^{2,7}]undec-5-en-5-yl]-1,1-dioxo-1,4-dihydro-1λ⁶-benzo[1,2,4]thiadiazin-7-yl}-methansulfonamid umfasst, und (ii) einer Zusammensetzung, die PSI-6130 umfasst.

14. Die Kombination zur Verwendung nach Anspruch 13, wobei das Subjekt ein Mensch ist.

15. Die Kombination zur Verwendung nach den Ansprüchen 13 oder 14, wobei bei der Verwendung bei der Behandlung oder Verbeugung der Hepatitis-C-Virus-Infektion die Zusammensetzungen getrennt verabreicht werden.

## Revendications

1. Procédé *in vitro* d'inhibition de la réplication du virus de l'hépatite C comprenant l'exposition du virus de l'hépatite C à une quantité thérapeutiquement efficace d'une composition comprenant N-{3-[(1*R*,2*S*,7*R*,8*S*)-3-(4-fluoro-benzyl)-6-hydroxy-4-oxo-3-aza-tricyclo[6.2.1.0^{2,7}]undéc-5-en-5-yl]-1,1-dioxo-1,4-dihydro-1λ⁶-benzo[1,2,4]thiadiazin-7-yl}-méthanesulfonamide, ou un sel ou un hydrate de celui-ci, et une composition comprenant un ou plusieurs composés antiviraux supplémentaires sélectionnés parmi le groupe constitué de : VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, et ANA773.

2. Procédé selon la revendication 1, dans lequel le virus de l'hépatite C est dans une cellule humaine.

3. Procédé selon la revendication 1 ou la revendication 2, dans lequel ledit un ou plusieurs composés antiviraux supplémentaires est PSI-6130.

4. Combinaison de (i) une composition comprenant *N*-{3-[(1*R*,2*S*,7*R*,8*S*)-3-(4-fluoro-benzyl)-6-hydroxy-4-oxo-3-aza-tricyclo[6.2.1.0^{2,7}]undéc-5-en-5-yl]-1,1-dioxo-1,4-dihydro-1λ⁶-benzo[1,2,4]thiadiazin-7-yl}-méthanesulfonamide, un sel ou un hydrate de celui-ci, et (ii) une composition comprenant un ou plusieurs composés antiviraux supplémentaires sélectionnés parmi le groupe constitué de : VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, et ANA773 pour une utilisation dans le traitement ou la prévention d'une infection au virus de l'hépatite C.

5. Combinaison pour utilisation selon la revendication 4, dans laquelle le sujet est un humain.

6. Combinaison pour utilisation selon la revendication 4 ou la revendication 5, dans laquelle dans l'utilisation dans le traitement ou la prévention d'une infection au virus de l'hépatite C, les compositions sont administrées séparément.

7. Combinaison pour utilisation selon les revendications 4 à 6, dans laquelle ledit un ou plusieurs composés antiviraux supplémentaires sont sélectionnés parmi MK-7009, TMC-435350, BI-201335, PF-00868554, IDX-184, RG7128, PSI-6130, BMS-790052, et ANA773.

8. Combinaison pour utilisation selon la revendication 7, dans laquelle ledit un ou plusieurs composés antiviraux supplémentaires est PSI-6130.

9. Composition pharmaceutiquement acceptable comprenant une quantité thérapeutiquement efficace d'une composition comprenant *N*-{3-[(1*R*,2*S*,7*R*,8*S*)-3-(4-fluoro-benzyl)-6-hydroxy-4-oxo-3-aza-tricyclo[6.2.1.0^{2,7}]undéc-5-en-5-yl]-1,1-dioxo-1,4-dihydro-1λ⁶-benzo[1,2,4]thiadiazin-7-yl}-méthanesulfonamide, ou un sel ou un hydrate de celui-ci, et un ou plusieurs composés antiviraux supplémentaires sélectionnés parmi le groupe constitué de : VBY-376, BMS-650032, MK-7009, TMC-435350, BI-201335, GS-9190, MK-3281, VCH-759, VCH-916, ABT-333, BMS-791325, PF-00868554, IDX-184, RG7128, PSI-6130, BMS-790052, et ANA773 et un support pharmaceutiquement acceptable.

10. Composition selon la revendication 9, dans laquelle ledit un ou plusieurs composés antiviraux supplémentaires sont sélectionnés parmi MK-7009, TMC-435350, BI-201335, PF-00868554, IDX-184, RG7128, PSI-6130, BMS-790052, et ANA773.

11. Composition selon la revendication 10, dans laquelle ledit un ou plusieurs composés antiviraux supplémentaires est PSI-6130.

12. Composition selon les revendications 9 à 11, dans laquelle le sel de M-{3-[(1*R*,2*S*,7*R*,8*S*)-3-(4-fluoro-benzyl)-6-hydroxy-4-oxo-3-aza-tricyclo[6.2.1.0^{2,7}]undéc-5-en-5-yl]-1,1-dioxo-1,4-dihydro-1λ⁶-benzo[1,2,4]thiadiazin-7-yl}-méthanesulfonamide est sélectionné parmi le groupe constitué de : *L*-arginine, *L*-lysine, hémi-magnésium, sodium et potassium.

13. Combinaison pour utilisation selon la revendication 4, dans laquelle la combinaison est une combinaison de (i) une composition comprenant *N*-{3-[(1*R*,2*S*,7*R*,8*S*)-3-(4-fluoro-benzyl)-6-hydroxy-4-oxo-3-aza-tricyclo[6.2.1.0^{2,7}]undéc-5-en-5-yl]-1,1-dioxo-1,4-dihydro-1λ⁶-benzo[1,2,4]thiadiazin-7-yl}-méthanesulfonamide, et (ii) une composition comprenant PSI-6130.

14. Combinaison pour utilisation selon la revendication 13, dans laquelle le sujet est un humain.

15. Combinaison pour utilisation selon la revendication 13 ou la revendication 14, dans laquelle dans l'utilisation dans le traitement ou la prévention d'une infection au virus de l'hépatite C, les compositions sont administrées séparément.
